# EUROPEAN PATENT APPLICATION

(11) **EP 4 582 545 A1**
(43) Date of publication of application: **09.07.2025**
(21) Application number: 23860337.7
(22) Date of filing: 29.08.2023
(51) Int. Cl.: C12N 15/13, C07K 16/28, C12N 5/0783

(54) **MONOBODY AND NK CELLS**

(30) Priority: 30.08.2022 JP 2022137288
(71) Applicant: GAIA BioMedicine Inc., Fukuoka-shi, Fukuoka 810-0041 (JP)
(72) Inventor: HARADA, Yui, Fukuoka-shi, Fukuoka 819-0395 (JP); YONEMITSU, Yoshikazu, Fukuoka-shi, Fukuoka 819-0395 (JP); MURAKAMI, Hiroshi, Nagoya-shi, Aichi 464-8601 (JP); HAYASHI, Gosuke, Nagoya-shi, Aichi 464-8601 (JP); FUJINO, Tomoshige, Nagoya-shi, Aichi 464-8601 (JP); TANAKA, Chihiro, Nagoya-shi, Aichi 464-8601 (JP)
(74) Representative: D Young & Co LLP
(86) International application number: PCT/JP2023/031204
(87) International publication number: WO 2024/048575

(57) **Abstract**

The present invention provides a novel substance that stably binds to a surface protein expressed on NK cells. The present invention provides a monobody that binds to a surface protein of an NK cell, wherein the monobody has an amino acid sequence of any one of (1) to (3): (1) an amino acid sequence represented by any one of SEQ ID NOs: A1 to A12; (2) an amino acid sequence given by deletion, substitution, and/or addition of one or more amino acids in an amino acid sequence represented by any one of SEQ ID NOs: A1 to A12 excluding arbitrary amino acids; and (3) an amino acid sequence having an identity of 80% or more with an amino acid sequence represented by any one of SEQ ID NOs: A1 to A12 excluding arbitrary amino acid sequences, wherein X¹, X², X³, X⁴, X⁵, and X⁶ in SEQ ID NOs: A1 to A12 each independently represent an arbitrary amino acid, and n1, n2, n3, n4, n5, and n6 each independently represent an arbitrary natural number.

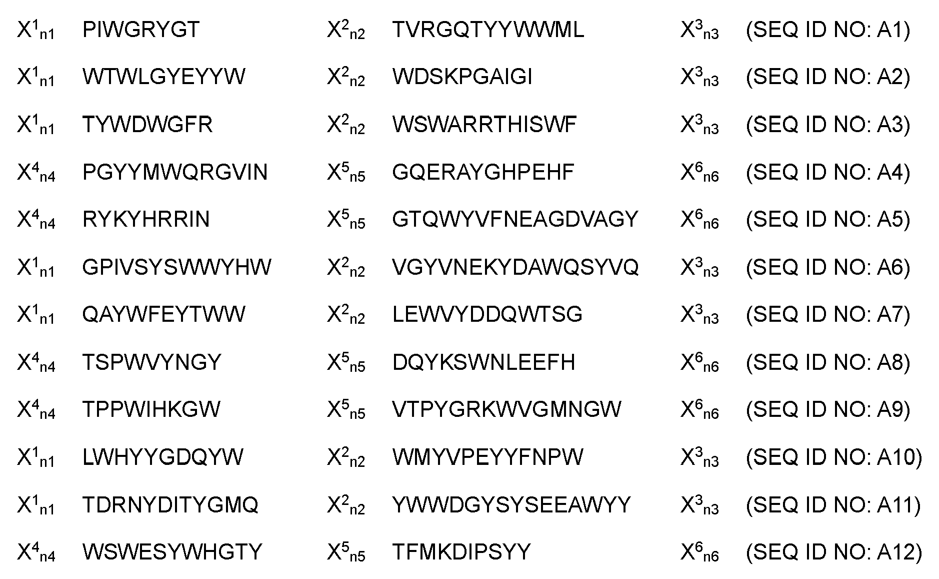

## Description

### Technical Field

The present invention relates to a monobody, in particular, a monobody capable of binding to NK cells.

### Background Art

Immunotherapies, which uses patient's own immunocompetence to damage cancer, are attracting attention as treatment methods for cancer. NK cell therapy, which uses natural killer (NK) cells, is known as one of immunotherapies. The NK cell therapy is a treatment method in which NK cells from peripheral blood mononuclear cells (PBMCs) of a patient are selectively grown and activated to enhance the damaging activity, and returned into the body of the patient.

An NK cell binds to the Fc region of an antibody through an Fcγ receptor to exert the ADCC (Antibody-Dependent Cellular Cytotoxicity) activity, and damages tumor cells. Accordingly, the NK cell therapy is expected to exert high synergistic effects when used in combination with an antibody drug targeting an antigen on tumor cells. In use in combination with an antibody drug, the antibody drug binds to an antigen, and activated NK cells and other immunocytes then accumulate to cause ADCC activity and ADCP (Antibody-Dependent Cellular Phagocytosis) activity, thereby killing tumor cells.

However, there are some normal cells having a target antigen expressed on tumor cells, and if an antibody drug binds to the antigen, immunocytes other than NK cells may attack such normal cells (on-target/off-tumor effect). As a method for suppressing the side effect, preparing a crosslinking structure that links an NK cell and an antibody drug to bind the cell and the antibody together in advance has been suggested (Patent Literature 1).

### Citation List

### Patent Literature

Patent Literature 1: International Publication No. WO 2022/004805

### Summary of Invention

### Technical Problem

In Patent Literature 1, however, an scFv is used for the NK binding domain of the crosslinking structure, and hence it is still needed to enhance the affinity between the NK binding domain and the target molecule of NK cell surfaces.

In such circumstances, an object of the present invention is to provide a novel substance that stably binds to a surface protein expressed on NK cells.

### Solution to Problem

The present inventors have diligently examined to solve the problem to find that a monobody having a specific amino acid sequence stably binds to a surface protein expressed on NK cells, eventually completing the present invention.

Specifically, the present invention is as follows.
[1] A monobody that binds to a surface protein of an NK cell, wherein the monobody has an amino acid sequence of any one of (1) to (3):
(1) an amino acid sequence represented by any one of SEQ ID NOs: A1 to A12;
(2) an amino acid sequence given by deletion, substitution, and/or addition of one or more amino acids in an amino acid sequence represented by any one of SEQ ID NOs: A1 to A12 excluding arbitrary amino acids; and
(3) an amino acid sequence having an identity of 80% or more with an amino acid sequence represented by any one of SEQ ID NOs: A1 to A12 excluding arbitrary amino acids, wherein
X¹, X², X³, X⁴, X⁵, and X⁶ in SEQ ID NOs: A1 to A12 are each independently an arbitrary amino acid, and n1, n2, n3, n4, n5, and n6 each independently represent an arbitrary natural number.

| | | | | | |
|---|---|---|---|---|---|
| X¹ₙ₁ | PIWGRYGT | X²ₙ₂ | TVRGQTYYWWML | X³ₙ₃ | (SEQ ID NO: A1) |
| X¹ₙ₁ | WTWLGYEYYW | X²ₙ₂ | WDSKPGAIGI | X³ₙ₃ | (SEQ ID NO: A2) |
| X¹ₙ₁ | TYWDWGFR | X²ₙ₂ | WSWARRTHISWF | X³ₙ₃ | (SEQ ID NO: A3) |
| X⁴ₙ₄ | PGYYMWQRGVIN | X⁵ₙ₅ | GQERAYGHPEHF | x⁶ₙ₆ | (SEQ ID NO: A4) |
| X⁴ₙ₄ | RYKYHRRIN | X⁵ₙ₅ | GTQWYVFNEAGDVAGY | x⁶ₙ₆ | (SEQ ID NO: A5) |
| X¹ₙ₁ | GPIVSYSWWYHW | X²ₙ₂ | VGYVNEKYDAWQSYVQ | X³ₙ₃ | (SEQ ID NO: A6) |
| X¹ₙ₁ | QAYWFEYTWW | X²ₙ₂ | LEWVYDDQWTSG | X³ₙ₃ | (SEQ ID NO: A7) |
| X⁴ₙ₄ | TSPWVYNGY | X⁵ₙ₅ | DQYKSWNLEEFH | x⁶ₙ₆ | (SEQ ID NO: A8) |
| X⁴ₙ₄ | TPPWIHKGW | X⁵ₙ₅ | VTPYGRKWVGMNGW | x⁶ₙ₆ | (SEQ ID NO: A9) |
| X¹ₙ₁ | LWHYYGDQYW | X²ₙ₂ | WMYVPEYYFNPW | X³ₙ₃ | (SEQ ID NO: A10) |
| X¹ₙ₁ | TDRNYDITYGMQ | X²ₙ₂ | YWWDGYSYSEEAWYY | X³ₙ₃ | (SEQ ID NO: A11) |
| X⁴ₙ₄ | WSWESYWHGTY | X⁵ₙ₅ | TFMKDIPSYY | X⁶ₙ₆ | (SEQ ID NO: A12) |

[2] The monobody according to [1], wherein the surface protein is at least one selected from the group consisting of NKp46, NKp30, and 2B4.
[3] The monobody according to [1] or [2], wherein, in the amino acid sequences represented by SEQ ID NOs: A1 to A12,
amino acid sequences represented by SEQ ID NOs: 1 to 12 are each present in a BC loop or CD loop, and
amino acid sequences represented by SEQ ID NOs: 13 to 24 are each present in an FG loop.

| | |
|---|---|
| PIWGRYGT | (SEQ ID NO: 1) |
| WTWLGYEYYW | (SEQ ID NO: 2) |
| TYWDWGFR | (SEQ ID NO: 3) |
| PGYYMWQRGVIN | (SEQ ID NO: 4) |
| RYKYHRRIN | (SEQ ID NO: 5) |
| GPIVSYSWWYHW | (SEQ ID NO: 6) |
| QAYWFEYTWW | (SEQ ID NO: 7) |
| TSPWVYNGY | (SEQ ID NO: 8) |
| TPPWIHKGW | (SEQ ID NO: 9) |
| LWHYYGDQYW | (SEQ ID NO: 10) |
| TDRNYDITYGMQ | (SEQ ID NO: 11) |
| WSWESYWHGTY | (SEQ ID NO: 12) |
| TVRGQTYYWWML | (SEQ ID NO: 13) |
| WDSKPGAIGI | (SEQ ID NO: 14) |
| WSWARRTHISWF | (SEQ ID NO: 15) |
| GQERAYGHPEHF | (SEQ ID NO: 16) |
| GTQWYVFNEAGDVAGY | (SEQ ID NO: 17) |
| VGYVNEKYDAWQSYVQ | (SEQ ID NO: 18) |
| LEWVYDDQWTSG | (SEQ ID NO: 19) |
| DQYKSWNLEEFH | (SEQ ID NO: 20) |
| VTPYGRKWVGMNGW | (SEQ ID NO: 21) |
| WMYVPEYYFNPW | (SEQ ID NO: 22) |
| YWWDGYSYSEEAWYY | (SEQ ID NO: 23) |
| TFMKDIPSYY | (SEQ ID NO: 24) |

[4] An NK cell, wherein the monobody according to any one of [1] to [3] is bound to a surface protein.

### Advantageous Effect of Invention

The present invention can provide a novel substance that stably binds to a surface protein expressed on NK cells.

### Brief Description of Drawings

[Figure 1] Figure 1 shows expression vectors constructed in Examples.
[Figure 2] Figure 2 shows monobody libraries.
[Figure 3] Figure 3 shows recovery rates in each round of monobody selection for a target mix (BC-FG: left, CD-FG: right).
[Figure 4] Figure 4 shows recovery rates in each round of monobody selection for different targets (BC-FG: top, CD-FG: bottom).
[Figure 5] Figure 5 shows measurement of dissociation constants for five clones that exhibited binding to NKp46.
[Figure 6] Figure 6 shows measurement of dissociation constants for four clones that exhibited binding to 2B4.
[Figure 7] Figure 7 shows measurement of dissociation constants for three clones that exhibited binding to NKp30.

### Description of Embodiments

The following describes modes for implementation of the present invention (hereinafter, referred to as "the present embodiment") in detail, but the present invention is not limited thereto, and various modifications without departing from the gist are acceptable.

The monobody of the present invention binds to a surface protein of an NK cell, and has an amino acid sequence of any one of (1) to (3). Here, X¹, X², X³, X⁴, X⁵, and X⁶ in SEQ ID NOs: A1 to A12 are each independently an arbitrary amino acid, and n1, n2, n3, n4, n5, and n6 each independently represent an arbitrary natural number.
(1) An amino acid sequence represented by any one of SEQ ID NOs: A1 to A12
(2) An amino acid sequence given by deletion, substitution, and/or addition of one or more amino acids in an amino acid sequence represented by any one of SEQ ID NOs: A1 to A12 excluding arbitrary amino acids
(3) An amino acid sequence having an identity of 80% or more with an amino acid sequence represented by any one of SEQ ID NOs: A1 to A12 excluding arbitrary amino acids

| | | | | | |
|---|---|---|---|---|---|
| X¹ₙ₁ | PIWGRYGT | X²ₙ₂ | TVRGQTYYWWML | X³ₙ₃ | (SEQ ID NO: A1) |
| X¹ₙ₁ | WTWLGYEYYW | X²ₙ₂ | WDSKPGAIGI | X³ₙ₃ | (SEQ ID NO: A2) |
| X¹ₙ₁ | TYWDWGFR | X²ₙ₂ | WSWARRTHISWF | X³ₙ₃ | (SEQ ID NO: A3) |
| X⁴ₙ₄ | PGYYMWQRGVIN | X⁵ₙ₅ | GQERAYGHPEHF | X⁶ₙ₆ | (SEQ ID NO: A4) |
| X⁴ₙ₄ | RYKYHRRIN | X⁵ₙ₅ | GTQWYVFNEAGDVAGY | X⁶ₙ₆ | (SEQ ID NO: A5) |
| X¹ₙ₁ | GPIVSYSWWYHW | X²ₙ₂ | VGYVNEKYDAWQSYVQ | X³ₙ₃ | (SEQ ID NO: A6) |
| X¹ₙ₁ | QAYWFEYTWW | X²ₙ₂ | LEWVYDDQWTSG | X³ₙ₃ | (SEQ ID NO: A7) |
| X⁴ₙ₄ | TSPWVYNGY | X⁵ₙ₅ | DQYKSWNLEEFH | X⁶ₙ₆ | (SEQ ID NO: A8) |
| X⁴ₙ₄ | TPPWIHKGW | X⁵ₙ₅ | VTPYGRKWVGMNGW | X⁶ₙ₆ | (SEQ ID NO: A9) |
| X¹ₙ₁ | LWHYYGDQYW | X²ₙ₂ | WMYVPEYYFNPW | X³ₙ₃ | (SEQ ID NO: A10) |
| X¹ₙ₁ | TORNYOITYGMQ | X²ₙ₂ | YWWDGYSYSEEAWYY | X³ₙ₃ | (SEQ ID NO: A11) |
| X⁴ₙ₄ | WSWESYWHGTY | X⁵ₙ₅ | TFMKDIPSYY | X⁶ₙ₆ | (SEQ ID NO: A12) |

Herein, the term "monobody" refers to an artificial protein that is produced by artificially mutating part of the sequence of a scaffold protein and has a molecular weight of about 10 kDa (more specifically, 7 kDa or more and 15 kDa or less). The molecular weight of the monobody of the present invention is, for example, 7 kDa or more and 15 kDa or less, and preferably 13.6 kDa or more and 14.0 kDa or less.

The number of amino acids constituting the monobody of the present invention is, for example, 50 or more and 230 or less, preferably 70 or more and 180 or less, and more preferably 90 or more and 150 or less. The number of amino acids may be in a range given by arbitrarily combining an upper limit value and lower limit value selected from those in the shown ranges.

The protein as a scaffold of the monobody of the present invention is not limited, and may be, for example, a part of fibronectin, or a part of human fibronectin, or a human fibronectin type III domain. That is, the monobody of the present invention is a product obtained by modifying the amino acid sequence of a part of fibronectin, a part of human fibronectin, or a human fibronectin type III domain. The monobody of the present invention may be a product obtained by modifying the sequence of a BC loop or CD loop of the human fibronectin type III domain, or a product obtained by modifying the sequence of an FG loop of the human fibronectin type III domain, or a product obtained by modifying the sequences of both a BC loop or CD loop and FG loop of the human fibronectin type III domain. The monobody of the present invention may be a polypeptide free of cysteine. Skeletons like the human fibronectin type III domain (FN3) are found in many animal proteins involved in ligand binding. The human fibronectin type III domain is similar in folding to the VH domain of immunoglobulin, and has a molecular weight of approximately 10 kDa, thus having high tissue-infiltrating ability. In addition, the human fibronectin type III domain has thermostability, and does not form any disulfide bond because of being cysteine-free.

The part derived from the human fibronectin type III domain in the monobody of the present invention may have an amino acid sequence represented by SEQ ID NO: 82, an amino acid sequence given by deletion, substitution, and/or addition of one or more amino acids in an amino acid sequence represented by SEQ ID NO: 82, or an amino acid sequence having an identity of 80% or more with an amino acid sequence represented by SEQ ID NO: 82. The BC loop of the human fibronectin type III domain may be a partial sequence corresponding to PAVT (SEQ ID NO: 83) in the amino acid sequence represented by SEQ ID NO: 82, the CD loop may be a partial sequence corresponding to GNSP (SEQ ID NO: 84) in the amino acid sequence represented by SEQ ID NO: 82, and the FG loop may be a partial sequence corresponding to GRGDSPASSK (SEQ ID NO: 85) in the amino acid sequence represented by SEQ ID NO: 82. The monobody of the present invention may be a product obtained by modifying a part of an amino acid sequence represented by SEQ ID NO: 82, an amino acid sequence given by deletion, substitution, and/or addition of one or more amino acids in an amino acid sequence represented by SEQ ID NO: 82, or an amino acid sequence having an identity of 80% or more with an amino acid sequence represented by SEQ ID NO: 82 and by inserting an amino acid sequence represented by any one of SEQ ID NOs: 1 to 24, an amino acid sequence given by deletion, substitution, and/or addition of one or more amino acids in an amino acid sequence represented by any one of SEQ ID NOs: 1 to 24, or an amino acid sequence having an identity of 80% or more with an amino acid sequence represented by any one of SEQ ID NOs: 1 to 24, as a result having an amino acid sequence of any one of (1) to (3). The modified site of the amino acid sequence derived from the human fibronectin type III domain may be a part of the amino acid sequence corresponding to the BC loop or CD loop of the human fibronectin type III domain, or a part of the amino acid sequence corresponding to the FG loop, or parts of the amino acid sequences corresponding to both the BC loop or CD loop and the FG loop.

LEVVAPTSISWDAPAVTVRYYRITYGETGGNSPVQEFTVPGSKSTATISGL
KPGVDYTITVYAVTGRGDSPASSKPISINYRTEIDKPSQ (SEQ ID NO: 82)

The monobody may have a plurality of β strands and loops connecting each strand to another. Although the sequence of the monobody may be modified at any site without limitation, it is preferable that the sequence of a loop region to be modified, and it is more preferable that the sequences of two or more loop regions to be modified.

The monobody may have seven or more β strands, and the sequences of at least two of the BC loop connecting the second and third strands, the CD loop connecting the third and fourth strands, and the FG loop connecting the sixth and seventh strands may be modified. Both of the sequences of the BC loop and the FG loop may be modified, and both of the sequences of the CD loop and the FG loop may be modified.

If the sequence of a loop region is modified, some or all of the amino acids constituting the loop may be modified. Here, the term "modification" for amino acid sequences means deleting, substituting, and/or adding one of more amino acids to an unmodified (i.e., natural) amino acid sequence.

The monobody of the present invention has an amino acid sequence of any one of (1) to (3) shown above.

In SEQ ID NOs: A1 to A12, X¹, X², X³, X⁴, X⁵, and X⁶ are each independently an arbitrary amino acid. Accordingly, if n1, n2, n3, n4, n5, and n6 are each a natural number of 2 or more in SEQ ID NOs: A1 to A12, X¹, X², X³, X⁴, X⁵, and X⁶ each form an amino acid sequence consisting of the arbitrary amino acids. However, if the monobody of the present invention has an amino acid sequence represented by SEQ ID NO: 82, an amino acid sequence given by deletion, substitution, and/or addition of one or more amino acids in an amino acid sequence represented by SEQ ID NO: 82, or an amino acid sequence having an identity of 80% or more with an amino acid sequence represented by SEQ ID NO: 82, X¹, X², X³, X⁴, X⁵, and X⁶ are each an amino acid corresponding to the amino acid sequence represented by SEQ ID NO: 82, the amino acid sequence given by deletion, substitution, and/or addition of one or more amino acids in an amino acid sequence represented by SEQ ID NO: 82, or the amino acid sequence having an identity of 80% or more with an amino acid sequence represented by SEQ ID NO: 82.

Herein, the meaning of the term "amino acid" includes not only natural amino acids but also artificial amino acid mutants and derivatives. Examples of amino acids include proteinogenic L-amino acids, non-proteinogenic amino acids, and chemically synthesized compounds having characteristics known in the art as the feature of amino acids.

The proteinogenic amino acids are, as represented as three-letter codes well known in the art, Arg, His, Lys, Asp, Glu, Ser, Thr, Asn, Gln, Cys, Gly, Pro, Ala, Ile, Leu, Met, Phe, Trp, Tyr, and Val. The proteinogenic amino acids are, as represented as one-letter codes well known in the art, R, H, K, D, E, S, T, N, Q, C, G, P, A, I, L, M, F, W, Y, and V.

The non-proteinogenic amino acids include natural or unnatural amino acids other than the proteinogenic amino acids.

The unnatural amino acids include: amino acids with the main chain structures differing from natural ones (e.g., α,α-disubstituted amino acids (e.g., α-methylalanine), N-alkylamino acids, D-amino acids, β-amino acids, γ-amino acids, δ-amino acids, and α-hydroxy acids); amino acids with the side chain structures differing from natural ones (e.g., norleucine and homohistidine); amino acids with the side chains having excessive methylene moieties (e.g., "homo"amino acids, homophenylalanine, and homohistidine); and amino acids with the side chains having substitution of a carboxylic acid functional group with a sulfonic acid group therein (e.g., cysteic acid).

X¹, X², X³, X⁴, X⁵, and X⁶ may be each independently a proteinogenic amino acid, and may be any one of the 19 proteinogenic amino acids with cysteine excluded. X⁶ and X³ may be identical.

In SEQ ID NOs: A1 to A12, n1, n2, n3, n4, n5, and n6 each independently represent an arbitrary natural number. Each of n1, n2, n3, n4, n5, and n6 may be, for example, 1 or more and 150 or less, and is preferably 3 or more and 100 or less.
n1 is preferably 5 or more and 50 or less, more preferably 10 or more and 30 or less, and still more preferably 13 or more and 25 or less.
n2 is preferably 10 or more and 100 or less, more preferably 30 or more and 70 or less, and still more preferably 40 or more and 60 or less.
n3 is preferably 3 or more and 60 or less, more preferably 5 or more and 55 or less, and still more preferably 10 or more and 50 or less.
n4 is preferably 10 or more and 80 or less, more preferably 20 or more and 70 or less, and still more preferably 25 or more and 50 or less.
n5 is preferably 10 or more and 80 or less, more preferably 20 or more and 70 or less, and still more preferably 25 or more and 50 or less.
n6 is preferably 3 or more and 60 or less, more preferably 5 or more and 55 or less, and still more preferably 10 or more and 50 or less. n6 and n3 may be equal.

In the amino acid sequence of (2), the phrase an amino acid sequence excluding arbitrary amino acids means an amino acid sequence other than amino acid sequence parts represented by X¹ₙ₁, X²ₙ₂, X³ₙ₃, X⁴ₙ₄, X⁵ₙ₅, and X⁶ₙ₆. Accordingly, the amino acid sequence of (2) is an amino acid sequence given by deletion, substitution, and/or addition of one or more amino acids in a sequence positioned between X¹ₙ₁ and X²ₙ₂ and/or a sequence positioned between X²ₙ₂ and X³ₙ₃, or a sequence positioned between X⁴ₙ₄ and X⁵ₙ₅ and/or a sequence positioned between X⁵ₙ₅ and X⁶ₙ₆ in an amino acid sequence represented by any one of SEQ ID NOs: A1 to A12.

Herein, the number of amino acids to be deleted, substituted, and/or added in "an amino acid sequence given by deletion, substitution, and/or addition of one or more amino acids in an amino acid sequence" may be any number of one or more without limitation. The term "more" in the phrase one or more amino acids means a natural number of 2 or more, and can be, for example, 2, 3, 4, 5, 6, 7, 8, 9, or 10.

The number of one or more may be 1 or more and 10 or less, and is preferably 1 or more and 8 or less, and more preferably 1 or more and 5 or less. The upper limit of the number of amino acids to be deleted, substituted, and/or added may be, for example, 10, 9, 8, 7, 6, 5, 4, 3, 2, or 1. The lower limit of the number of amino acids to be deleted, substituted, and/or added may be, for example, 2, 3, or 4. The number of amino acids to be deleted, substituted, and/or added may be in a range given by arbitrarily combining an upper limit value and lower limit value selected from those in the shown ranges.

In the amino acid sequence of (3), the phrase an amino acid sequence excluding arbitrary amino acids means an amino acid sequence other than amino acid sequence parts represented by X¹ₙ₁, X²ₙ₂, X³ₙ₃, X⁴ₙ₄, X⁵ₙ₅, and X⁶ₙ₆. Accordingly, the amino acid sequence of (3) is an amino acid sequence having an identity of 80% or more with a sequence positioned between X¹ₙ₁ and X²ₙ₂ and/or a sequence positioned between X²ₙ₂ and X³ₙ₃, or a sequence positioned between X⁴ₙ₄ and X⁵ₙ₅ and/or a sequence positioned between X⁵ₙ₅ and X⁶ₙ₆ in an amino acid sequence represented by any one of SEQ ID NOs: A1 to A12.

The phrase "an amino acid sequence having an identity of 80% or more with an amino acid sequence" in the present specification will be described with reference to the situation "having an identity of Y% or more with amino acid sequence X"; this phrase means that when the amino acid sequence having identity is aligned with amino acid sequence X to achieve the maximum matching (alignment), the proportion of the number of matching amino acid residues to the total number of the amino acids contained in the amino acid sequence having identity is Y% or more.

The identity is only required to be 80% or more, and may be 85% or more, 90% or more, 95% or more, 97% or more, 98% or more, or 99% or more. The upper limit of the identity is not limited, and may be 99%, 98%, 97%, or 95%. The identity may be 100%.

Search and analysis on the identity of amino acid sequences can be performed with an algorithm or program well known to those skilled in the art (e.g., BLASTN, BLASTP, BLASTX, ClustalW). Those skilled in the art could properly set parameters in using such a program, and default parameters of a program may be used.

For any one of the amino acid sequences of (1) to (3), it is preferable that amino acid sequences represented by SEQ ID NOs: 1 to 12 be each present in the BC loop or CD loop and amino acid sequences represented by SEQ ID NOs: 13 to 24 be each present in the FG loop in the amino acid sequences represented by SEQ ID NOs: A1 to A12. It is particularly preferable that amino acid sequences represented by SEQ ID NOs: 1 to 3, 6 to 7, and 10 to 11 be each present in the BC loop and amino acid sequences represented by SEQ ID NOs: 4 to 5, 8 to 9, and 12 be each present in the CD loop.

| | |
|---|---|
| PIWGRYGT | (SEQ ID NO: 1) |
| WTWLGYEYYW | (SEQ ID NO: 2) |
| TYWDWGFR | (SEQ ID NO: 3) |
| PGYYMWQRGVIN | (SEQ ID NO: 4) |
| RYKYHRRIN | (SEQ ID NO: 5) |
| GPIVSYSWWYHW | (SEQ ID NO: 6) |
| QAYWFEYTWW | (SEQ ID NO: 7) |
| TSPWVYNGY | (SEQ ID NO: 8) |
| TPPWIHKGW | (SEQ ID NO: 9) |
| LWHYYGDQYW | (SEQ ID NO: 10) |
| TDRNYDITYGMQ | (SEQ ID NO: 11) |
| WSWESYWHGTY | (SEQ ID NO: 12) |
| TVRGQTYYWWML | (SEQ ID NO: 13) |
| WDSKPGAIGI | (SEQ ID NO: 14) |
| WSWARRTHISWF | (SEQ ID NO: 15) |
| GQERAYGHPEHF | (SEQ ID NO: 16) |
| GTQWYVFNEAGDVAGY | (SEQ ID NO: 17) |
| VGYVNEKYDAWQSYVQ | (SEQ ID NO: 18) |
| LEWVYDDQWTSG | (SEQ ID NO: 19) |
| DQYKSWNLEEFH | (SEQ ID NO: 20) |
| VTPYGRKWVGMNGW | (SEQ ID NO: 21) |
| WMYVPEYYFNPW | (SEQ ID NO: 22) |
| YWWDGYSYSEEAWYY | (SEQ ID NO: 23) |
| TFMKDIPSYY | (SEQ ID NO: 24) |

In the present invention, the monobody may be:
(1) a monobody in which an amino acid sequence represented by any one of SEQ ID NOs: 1 to 12 is present in the BC loop or CD loop and an amino acid sequence represented by any one of SEQ ID NOs: 13 to 24 is present in the FG loop, and in this case the monobody has any one of the combinations:
   SEQ ID NO: 1 and SEQ ID NO: 13;
   SEQ ID NO: 2 and SEQ ID NO: 14;
   SEQ ID NO: 3 and SEQ ID NO: 15;
   SEQ ID NO: 4 and SEQ ID NO: 16;
   SEQ ID NO: 5 and SEQ ID NO: 17;
   SEQ ID NO: 6 and SEQ ID NO: 18;
   SEQ ID NO: 7 and SEQ ID NO: 19;
   SEQ ID NO: 8 and SEQ ID NO: 20;
   SEQ ID NO: 9 and SEQ ID NO: 21;
   SEQ ID NO: 10 and SEQ ID NO: 22;
   SEQ ID NO: 11 and SEQ ID NO: 23; and
   SEQ ID NO: 12 and SEQ ID NO: 24.

The monobody may be:
(2) a monobody having an amino acid sequence given by deletion, substitution, and/or addition of one or more amino acids in an amino acid sequence represented by any one of the combinations of SEQ ID NOs: 1 to 12 and SEQ ID NOs: 13 to 24.

The monobody may be:
(3) a monobody having an amino acid sequence having an identity of 80% or more with an amino acid sequence represented by any one of the combinations of SEQ ID NOs: 1 to 12 and SEQ ID NOs: 13 to 24.

Herein, in an amino acid sequence represented by any one of SEQ ID NOs: A1 to A12, the phrase an amino acid sequence excluding arbitrary amino acids means an amino acid sequence other than amino acid sequences each represented with any one of X¹, X², X³, X⁴, X⁵, and X⁶. Specifically, in each of SEQ ID NOs: A1 to A12, the amino acid sequence excluding arbitrary amino acids is a pair of the amino acid sequence correspondingly selected from the amino acid sequences represented by SEQ ID NOs: 1 to 12 and the amino acid sequence correspondingly selected from the amino acid sequences represented by SEQ ID NOs: 13 to 24.

In the case of SEQ ID NO: A1 as an example, the amino acid sequences of SEQ ID NO: 1 and SEQ ID NO: 13 correspond to an amino acid sequence excluding the arbitrary amino acids.

(2) The phrase an amino acid sequence given by deletion, substitution, and/or addition of one or more amino acids in an amino acid sequence represented by any one of SEQ ID NOs: A1 to A12 excluding arbitrary amino acids means, as described for the case of SEQ ID NO: A1 as an example, an amino acid sequence given by deletion, substitution, and/or addition of one or more amino acids in the amino acid sequences of SEQ ID NO: 1 and SEQ ID NO: 13. This amino acid sequence may be an amino acid sequence with deletion, substitution, and/or addition of one or more amino acids in the amino acid sequence of SEQ ID NO: 1 (no mutation in SEQ ID NO: 13), or an amino acid sequence with deletion, substitution, and/or addition of one or more amino acids in the amino acid sequence of SEQ ID NO: 13 (no mutation in SEQ ID NO: 1), or an amino acid sequence with deletion, substitution, and/or addition of one or more amino acids in the amino acid sequences of SEQ ID NO: 1 and SEQ ID NO: 13 (mutated in both SEQ ID NO: 1 and SEQ ID NO: 13).

(3) The phrase an amino acid sequence having an identity of 80% or more with an amino acid sequence represented by any one of SEQ ID NOs: A1 to A12 excluding arbitrary amino acids means, as described for the case of SEQ ID NO: A1 as an example, an amino acid sequence having an identity of 80% or more with the amino acid sequences of SEQ ID NO: 1 and SEQ ID NO: 13. This amino acid sequence may be an amino acid sequence in which the part corresponding to the amino acid sequence of SEQ ID NO: 1 has an identity of 80% or more therewith (no mutation in SEQ ID NO: 13), or an amino acid sequence in which the part corresponding to the amino acid sequence of SEQ ID NO: 13 has an identity of 80% or more therewith (no mutation in SEQ ID NO: 1), or an amino acid sequence in which the parts corresponding to the amino acid sequences of SEQ ID NO: 1 and SEQ ID NO: 13 have an identity of 80% or more therewith (mutated in both SEQ ID NO: 1 and SEQ ID NO: 13).

For each of SEQ ID NOs: A2 to A12, the description of SEQ ID NO: A1 is applied to the combination of amino acid sequences specific to each sequence, likewise.

The amino acid sequence represented by X¹ₙ₁ in SEQ ID NOs: A1 to A12 is not limited, and preferably an amino acid sequence represented by SEQ ID NO: 25, an amino acid sequence given by deletion, substitution, and/or addition of one or more amino acids in an amino acid sequence represented by SEQ ID NO: 25, or an amino acid sequence having an identity of 80% or more with an amino acid sequence represented by SEQ ID NO: 25.

The amino acid sequence represented by X¹ₙ₁ in SEQ ID NOs: A1 to A12 is more preferably an amino acid sequence represented by SEQ ID NO: A25, or an amino acid sequence given by substitution of one or more amino acids in an amino acid sequence represented by SEQ ID NO: A25 and having an identity of 80% or more with the amino acid sequence represented by SEQ ID NO: A25. Here, X⁷ in SEQ ID NO: A25 each independently represents an arbitrary amino acid, and n7 is a natural number of 1 or more and 10 or less, and preferably a natural number of 1 or more and 5 or less. X⁷ₙ₇ may contain an amino acid corresponding to a start codon (e.g., methionine) and an insertion sequence. The amino acids to be substituted in the amino acid sequence represented by SEQ ID NO: A25 may be those other than E at the fifth position, S at the seventh position, I at the 11th position, and Q at the 12th position, each position being counted from the N terminus with X⁷ₙ₇ excluded.

The amino acid sequence represented by X¹ₙ₁ in SEQ ID NOs: A1 to A12 is still more preferably an amino acid sequence represented by SEQ ID NO: 26, or an amino acid sequence given by substitution of one or more amino acids in an amino acid sequence represented by SEQ ID NO: 26 and having an identity of 80% or more with the amino acid sequence represented by SEQ ID NO: 26. The amino acids to be substituted in the amino acid sequence represented by SEQ ID NO: 26 may be those other than E at the ninth position, S at the 11th position, I at the 15th position, and Q at the 16th position, each position being counted from the N terminus.

The amino acid sequence represented by X²ₙ₂ in SEQ ID NOs: A1 to A12 is not limited, and preferably an amino acid sequence represented by SEQ ID NO: 27, an amino acid sequence given by deletion, substitution, and/or addition of one or more amino acids in an amino acid sequence represented by SEQ ID NO: 27, or an amino acid sequence having an identity of 80% or more with an amino acid sequence represented by SEQ ID NO: 27.

The amino acid sequence represented by X³ₙ₃ in SEQ ID NOs: A1 to A12 is not limited, and preferably contains an amino acid sequence represented by SEQ ID NO: 28, an amino acid sequence given by deletion, substitution, and/or addition of one or more amino acids in an amino acid sequence represented by SEQ ID NO: 28, or an amino acid sequence having an identity of 80% or more with an amino acid sequence represented by SEQ ID NO: 28.

The amino acid sequence represented by X³ₙ₃ in SEQ ID NOs: A1 to A12 is more preferably an amino acid sequence represented by SEQ ID NO: A28, or an amino acid sequence given by substitution of one or more amino acids in an amino acid sequence represented by SEQ ID NO: A28 and having an identity of 80% or more with the amino acid sequence represented by SEQ ID NO: A28. Here, X⁸ in SEQ ID NO: A28 each independently represents an arbitrary amino acid, and n8 is an integer of 0 or more and 40 or less, and preferably an integer of 0 or more and 30 or less. X⁸ₙ₈ may contain a linker sequence.

The amino acid sequence represented by X⁴ₙ₄ in SEQ ID NOs: A1 to A12 is not limited, and preferably an amino acid sequence represented by SEQ ID NO: 29, an amino acid sequence given by deletion, substitution, and/or addition of one or more amino acids in an amino acid sequence represented by SEQ ID NO: 29, or an amino acid sequence having an identity of 80% or more with an amino acid sequence represented by SEQ ID NO: 29.

The amino acid sequence represented by X⁴ₙ₄ in SEQ ID NOs: A1 to A12 is more preferably an amino acid sequence represented by SEQ ID NO: A29, or an amino acid sequence given by substitution of one or more amino acids in an amino acid sequence represented by SEQ ID NO: A29 and having an identity of 80% or more with the amino acid sequence represented by SEQ ID NO: A29. Here, Z and X⁷ in SEQ ID NO: A29 each independently represent an arbitrary amino acid, and n7 is a natural number of 1 or more and 10 or less, and preferably a natural number of 1 or more and 5 or less. X⁷ₙ₇ may contain an amino acid corresponding to a start codon (e.g., methionine) and an insertion sequence. ZIZ at the 26th to 28th positions from the N terminus with X⁷ₙ₇ excluded in the amino acid sequence represented by SEQ ID NO: A29 may be DIY in SEQ ID NO: A4, VIS in SEQ ID NO: A5, GIY in SEQ ID NO: A8, EIL in SEQ ID NO: A9, and VIT in SEQ ID NO: A12. The amino acids to be substituted in the amino acid sequence represented by SEQ ID NO: A29 may be those other than E at the fifth position, S at the seventh position, I at the 11th position, Q at the 12th position, and ZIZ at the 26th to 28th positions, each position being counted from the N terminus with X⁷ₙ₇ excluded.

The amino acid sequence represented by X⁴ₙ₄ in SEQ ID NOs: A1 to A12 is still more preferably an amino acid sequence represented by SEQ ID NO: 30, or an amino acid sequence given by substitution of one or more amino acids in an amino acid sequence represented by SEQ ID NO: 30 and having an identity of 80% or more with the amino acid sequence represented by SEQ ID NO: 30. Here, Z in SEQ ID NO: 30 each independently represents an arbitrary amino acid. ZIZ at the 30th to 32nd positions from the N terminus in the amino acid sequence represented by SEQ ID NO: 30 may be DIY in SEQ ID NO: A4, VIS in SEQ ID NO: A5, GIY in SEQ ID NO: A8, EIL in SEQ ID NO: A9, and VIT in SEQ ID NO: A12. The amino acids to be substituted in the amino acid sequence represented by SEQ ID NO: 30 may be those other than E at the ninth position, S at the 11th position, I at the 15th position, Q at the 16th position, and ZIZ at the 30th to 32nd positions, each position being counted from the N terminus.

The amino acid sequence represented by X⁵ₙ₅ in SEQ ID NOs: A1 to A12 is not limited, and preferably an amino acid sequence represented by SEQ ID NO: 31, an amino acid sequence given by deletion, substitution, and/or addition of one or more amino acids in an amino acid sequence represented by SEQ ID NO: 31, or an amino acid sequence having an identity of 80% or more with an amino acid sequence represented by SEQ ID NO: 31. Here, Z in SEQ ID NO: 31 each independently represents an arbitrary amino acid.

The amino acid sequence represented by X⁵ₙ₅ in SEQ ID NOs: A1 to A12 is more preferably an amino acid sequence represented by SEQ ID NO: 31, or an amino acid sequence given by substitution of one or more amino acids in an amino acid sequence represented by SEQ ID NO: 31 and having an identity of 80% or more with the amino acid sequence represented by SEQ ID NO: 31. Z at the third position from the N terminus in the amino acid sequence represented by SEQ ID NO: 31 may be A in SEQ ID NO: A4, G in SEQ ID NO: A5, E in SEQ ID NO: A8, Y in SEQ ID NO: A9, and Q in SEQ ID NO: A12. The amino acids to be substituted in the amino acid sequence represented by SEQ ID NO: 31 may be those other than Z at the third position counted from the N terminus.

The amino acid sequence represented by X⁶ₙ₆ is not limited, and may be the same as the amino acid sequence represented by X³ₙ₃.

The number of amino acids to be deleted, substituted, and/or added or the number of amino acids to be substituted in each of the acid sequences represented by SEQ ID NOs: 25 to 31, A25, A28, and A29 is 1 or more and 10 or less, and preferably 1 or more and 8 or less. The upper limit of the number of amino acids to be deleted, substituted, and/or added or the number of amino acids to be substituted may be, for example, 10, 9, 8, 7, 6, 5, 4, 3, 2, or 1. The lower limit of the number of amino acids to be deleted, substituted, and/or added or the number of amino acids to be substituted may be, for example, 2, 3, or 4. The number of amino acids to be deleted, substituted, and/or added or the number of amino acids to be substituted may be in a range given by arbitrarily combining an upper limit value and lower limit value selected from those in the shown ranges.

| | | |
|---|---|---|
| LEVVAPTSISWDA | (SEQ ID NO: 25) | (X¹ₙ₁) |
| X⁷ₙ₇LEVVEASPTSIQISWDA | (SEQ ID NO: A25) | (X¹ₙ₁) |
| MPNRLEVVEASPTSIQISWDA | (SEQ ID NO: 26) | (X¹ₙ₁) |
| VRYYRITYGETGGNSPVQEFTVPGSKSTATISGLKPG VDYTITVYAVT | (SEQ ID NO: 27) | (X²ₙ₂) |
| PISINYRTEIDKPSQ | (SEQ ID NO: 28) | (X³ₙ₃) |
| PISINYRTEIDKPSQX⁸ ₙ₈ | (SEQ ID NO: A28) | (X³ₙ₃) |
| LEVVAPTSISWDAPAVTVRYYRITYGETG | (SEQ ID NO: 29) | (X⁴ₙ₄) |
| X⁷ₙ₇LEVVEASPTSIQISWDAPAVTVRYYZIZYGETG | (SEQ ID NO: A29) | (X⁴ₙ₄) |
| MPNRLEVVEASPTSIQISWDAPAVTVRYYZIZYGETG | (SEQ ID NO: 30) | (X⁴ₙ₄) |
| VQZFTVPGSKSTATISGLKPGVDYTITVYAVT | (SEQ ID NO: 31) | (X⁵ₙ₅) |

The amino acid sequences represented by SEQ ID NOs: A4 to A5, A8 to A9, and A12 are preferably SEQ ID NOs: A32 to A35, and more preferably SEQ ID NOs: A36 to A41. The amino acid sequences represented by SEQ ID NOs: A1 to A3, A6 to A7, and A10 to A11 are preferably SEQ ID NOs: A42 to A48. In SEQ ID NOs: A32 to A48, loop regions modified from the human fibronectin type III domain are each indicated by an underline. X⁷ₙ₇ in SEQ ID NOs: A32 to A48 may contain an amino acid corresponding to a start codon (e.g., methionine) and an insertion sequence, and X⁸ₙ₈ may contain a linker sequence. In SEQ ID NOs: A32 to A48, Z, X⁷, and X⁸ each independently represent an arbitrary amino acid, n7 represents a natural number of 1 or more and 10 or less, and n8 represents an integer of 0 or more and 40 or less. n7 is preferably an integer of 1 or more and 5 or less, and n8 is preferably an integer of 0 or more and 30 or less.

X7n7LEVVEASPTSIQISWDAPAVTVRYYZIZYGETG
PGYYMWQRGVIN VQZFTVPGSKSTATISGLKPGVDYTITVYAVT (SEQ ID NO: A32)
GQERAYGHPEHF PISINYRTEIDKPSQX8n8

X^7_n7LEVVEASPTSIQISWDAPAVTVRYYZIZYGETG RYKYHRRIN
VQZFTVPGSKSTATISGLKPGVDYTITVYAVT (SEQ ID NO: A33)
GTQWYVFNEAGDVAGY PISINYRTEIDKPSQX^8_n8

X^7_n7LEVVEASPTSIQISWDAPAVTVRYYZIZYGETG TSPWVYNGY
VQZFTVPGSKSTATISGLKPGVDYTITVYAVT DQYKSWNLEEFH (SEQ ID NO: A34)
PISINYRTEIDKPSQX^8_n8

X7n7LEVVEASPTSIQISWDAPAVTVRYYZIZYGETG TPPWIHKGW
VQZFTVPGSKSTATISGLKPGVDYTITVYAVT (SEQ ID NO: A35)
VTPYGRKWVGMNGW PISINYRTEIDKPSQX8n8

X7n7LEVVEASPTSIQISWDAPAVTVRYYZIZYGETG
WSWESYWHGTY VQZFTVPGSKSTATISGLKPGVDYTITVYAVT (SEQ ID NO: A36)
TFMKDIPSYY PISINYRTEIDKPSQX8n8

X⁷n7LEVVEASPTSIQISWDAPAVTVRYYDIYYGETG
PGYYMWQRGVIN VQAFTVPGSKSTATISGLKPGVDYTITVYAVT (SEQ ID NO: A37)
GQERAYGHPEHF PISINYRTEIDKPSQX⁸n8

X⁷n7LEVVEASPTSIQISWDAPAVTVRYYVISYGETG RYKYHRRIN
VQGFTVPGSKSTATISGLKPGVDYTITVYAVT (SEQ ID NO: A38)
GTQWYVFNEAGDVAGY PISINYRTEIDKPSQX⁸n8

X^7_n7LEVVEASPTSIQISWDAPAVTVRYYGIYYGETG TSPWVYNGY
VQEFTVPGSKSTATISGLKPGVDYTITVYAVT DQYKSWNLEEFH (SEQ ID NO: A39)
PISINYRTEIDKPSQX^8_n8

X^7_n7LEVVEASPTSIQISWDAPAVTVRYYEILYGETG TPPWIHKGW
VQYFTVPGSKSTATISGLKPGVDYTITVYAVT (SEQ ID NO: A40)
VTPYGRKWVGMNGW PISINYRTEIDKPSQX^8_n8

X^7_n7LEVVEASPTSIQISWDAPAVTVRYYVITYGETG
WSWESYWHGTY VQQFTVPGSKSTATISGLKPGVDYTITVYAVT (SEQ ID NO: A41)
TFMKDIPSYY PISINYRTEIDKPSQX^8_n8

X7n7LEVVEASPTSIQISWDA PIWGRYGT
VRYYRITYGETGGNSPVQEFTVPGSKSTATISGLKPGVDYTITVYAVT
TVRGQTYYWWML PISINYRTEIDKPSQX8n8
(SEQ ID NO:
A42)

X⁷n7LEVVEASPTSIQISWDA WTWLGYEYYW
VRYYRITYGETGGNSPVQEFTVPGSKSTATISGLKPGVDYTITVYAVT
WDSKPGAIGI PISINYRTEIDKPSQX⁸n8
(SEQ ID NO:
A43)

X^7_n7LEVVEASPTSIQISWDA TYWDWGFR
VRYYRITYGETGGNSPVQEFTVPGSKSTATISGLKPGVDYTITVYAVT
WSWARRTHISWF PISINYRTEIDKPSQX^8_n8
(SEQ ID NO:
A44)

X⁷n7LEVVEASPTSIQISWDA GPIVSYSWWYHW
VRYYRITYGETGGNSPVQEFTVPGSKSTATISGLKPGVDYTITVYAVT
VGYVNEKYDAWQSYVQ PISINYRTEIDKPSQX⁸n8
(SEQ ID NO:
A45)

X7n7LEVVEASPTSIQISWDA QAYWFEYTWW
VRYYRITYGETGGNSPVQEFTVPGSKSTATISGLKPGVDYTITVYAVT
LEWVYDDQWTSG PISINYRTEIDKPSQX8n8
(SEQ ID NO:
A46)

X7n7LEVVEASPTSIQISWDA LWHYYGDQYW
VRYYRITYGETGGNSPVQEFTVPGSKSTATISGLKPGVDYTITVYAVT
WMYVPEYYFNPW PISINYRTEIDKPSQX8n8
(SEQ ID NO:
A47)

X7n7LEVVEASPTSIQISWDA TDRNYDITYGMQ
VRYYRITYGETGGNSPVQEFTVPGSKSTATISGLKPGVDYTITVYAVT
YWWDGYSYSEEAWYY PISINYRTEIDKPSQX8n8
(SEQ ID NO:
A48)

Although SEQ ID NOs: A32 to A48 each contain an amino acid sequence of any one of combinations shown below, the monobody of the present invention may be a monobody having an amino acid sequence given by deletion, substitution, and/or addition of one or more amino acids in an amino acid sequence excluding an amino acid sequence of any one of combinations shown below, or a monobody having an amino acid sequence such that an amino acid sequence excluding an amino acid sequence of any one of combinations shown below has an identity of 80% or more with the corresponding amino acid sequence:
SEQ ID NO: 1 and SEQ ID NO: 13;
SEQ ID NO: 2 and SEQ ID NO: 14;
SEQ ID NO: 3 and SEQ ID NO: 15;
SEQ ID NO: 4 and SEQ ID NO: 16;
SEQ ID NO: 5 and SEQ ID NO: 17;
SEQ ID NO: 6 and SEQ ID NO: 18;
SEQ ID NO: 7 and SEQ ID NO: 19;
SEQ ID NO: 8 and SEQ ID NO: 20;
SEQ ID NO: 9 and SEQ ID NO: 21;
SEQ ID NO: 10 and SEQ ID NO: 22
SEQ ID NO: 11 and SEQ ID NO: 23; and
SEQ ID NO: 12 and SEQ ID NO: 24.

The monobody of the present invention, with an amino acid sequence of any one of SEQ ID NOs: A32 to A48, may be a monobody having an amino acid sequence such that an amino acid sequence excluding an amino acid sequence of any one of the combinations is the same as the corresponding amino acid sequence, or having an amino acid sequence given by deletion, substitution, and/or addition of one or more amino acids in an amino acid sequence excluding an amino acid sequence of any one of the combinations, or having an amino acid sequence such that an amino acid sequence excluding an amino acid sequence of any one of the combinations has an identity of 80% or more with the corresponding amino acid sequence, wherein the monobody may have an amino acid sequence of any one of the combinations or an amino acid sequence given by mutation or the like in an amino acid sequence of any one of the combination.

As any one of the combinations is represented as SEQ ID NO: X1 and SEQ ID NO: X2, the amino acid sequence with mutation or the like in an amino acid sequence of the corresponding combination may be an amino acid sequence with mutation or the like in the part corresponding to SEQ ID NO: X1, or an amino acid sequence with mutation or the like in the part corresponding to SEQ ID NO: X2, or an amino acid sequence with mutation or the like in the parts corresponding to both SEQ ID NOs: X1 and X2.

The amino acid sequence with mutation or the like in the part corresponding to SEQ ID NO: X1 may be an amino acid sequence with deletion, substitution, and/or addition of one or more amino acids in the part corresponding to SEQ ID NO: X1, and may be an amino acid sequence in which the part corresponding to SEQ ID NO: X1 has an identity of 80% or more therewith.

The amino acid sequences with mutation or the like in the part corresponding to SEQ ID NO: X2 may be an amino acid sequence with deletion, substitution, and/or addition of one or more amino acids in the part corresponding to SEQ ID NO: X2, and may be an amino acid sequence in which the part corresponding to SEQ ID NO: X2 has an identity of 80% or more therewith.

Any combination of SEQ ID NO: X1 and SEQ ID NO: X2 is contemplated.

The amino acid sequence with mutation or the like in the parts corresponding to SEQ ID NO: X1 and SEQ ID NO: X2 is preferably an amino acid sequence with substitution of one or more amino acids in each of the parts corresponding to SEQ ID NO: X1 and SEQ ID NO: X2, or an amino acid sequence in which the parts corresponding to SEQ ID NO: X1 and SEQ ID NO: X2 have an identity of 80% or more therewith.

A tag sequence and/or a fusion protein may be further bound to the monobody of the present invention. The binding site is not limited, and may be, for example, the C terminus of the monobody or X⁸ₙ₈ in any one of SEQ ID NOs: A32 to A48.

The monobody of the present invention may be produced with a conventionally known method such as translation synthesis using a cell system or cell-free system and chemical synthesis, and is preferably produced with translation synthesis using a cell system. With the translation synthesis using a cell system, for example, a monobody having a desired amino acid sequence may be produced by introducing an insert DNA designed as appropriate for the desired amino acid sequence into a proper vector and cloning with a proper method, then expressing the DNA with proper cells such as competent cells, and purifying the DNA. More specifically, the monobody may be produced with a method described in Examples.

The monobody of the present invention binds to a surface protein of NK cells.

NK cells are large granular lymphocytes that are expressing none of the T-cell receptor (TCR), CD3, which is a universal marker for T cells, and the B-cell receptor, which is a membrane immunoglobulin, and are normally CD16-positive and CD56-positive in humans. Those skilled in the art could easily determine whether a cell is an NK cell or not, for example, on the basis of expression patterns of cell surface markers. NK cells have cytotoxic activity, and the presence or absence and degree of the cytotoxic activity can be determined with various known methods.

Unless otherwise stated, NK cells in the present invention include the common NK cells. NK cells include peripheral blood NK cells, cord blood NK cells, primary NK cells, cultured NK cells, and highly active NK cells, and NK cells and NK-like cells of [1], [2], [3], and [4] in the following.

[1] NK cells having the following features of (i) and (ii):
   (i) being CD16-positive, having high expression of CD56, and being CD57-negative; and
   (ii) being NKG2C-positive, being NKG2A-negative to having low expression thereof, and being CD94-positive.

The highly active NK cells of [1] may have high expression of CD16. Irrespective of having high expression of CD16 or not, the highly active NK cells of [1] may further have the following feature:
(iii) the cytotoxic activity is 50% or more when the NK cells as effector cells (E) and K562 cells as target cells (T) are cocultured at a mixing ratio (E:T) of 1:1.

The highly active NK cells of [1] can be represented as follows:
NK cells having the features of (i) and (iii), wherein the NK cells are obtained by removing CD3-positive cells from peripheral blood monocytes derived from a healthy individual by using CD3 beads (e.g., CliniMACS CD3, Miltenyi Biotec, catalog No. 130-017-601), an LD column (e.g., Miltenyi Biotec, catalog No. 130-042-901), and separation buffer (e.g., PBS containing 0.5% human type AB serum (inactivated) and 2 mM EDTA) to give a cell population, and culturing the cell population in a proper medium (e.g., COSMEDIUM 008 supplemented with 5% human type AB serum (inactivated)) for 14 days. Reference can be made to Japanese Patent Laid-Open No. 2018-193303 for details of the features of the highly active NK cells of [1] and a more specific production method therefor.

[2] The following cells:
cells being CCR5-positive, CCR6-positive, and CXCR3-positive, and being CD3-negative.

The cells of [2] may further have high expression of CD11c.

The cells of [2] can be represented as follows:
cells being CCR5-positive, CCR6-positive, CXCR3-positive, Integrin α1-positive, Integrin α3-positive, and Integrin β3-negative, and being CD3-negative; alternatively, cells being CCR5-positive, CCR6-positive, and CXCR3-positive, having high expression of CD11a, and having high expression of CD11c, and being CD3-negative (the high-expression character is determined by comparison with expression in a population of NK cells obtained from peripheral blood substantially without being cultured).

The cells of [2] exhibit extremely high cytotoxic activity to solid cancer with the formation of a tumor mass. Reference can be made to Japanese Patent Laid-Open No. 2019-170176 for details of the features the cells of [2] and a more specific production method therefor.

[3] Highly active NK cells obtainable with the following method:
to monocytes obtained from fresh peripheral blood or a frozen apheresis blood product, CD3 beads (e.g., CliniMACS CD3, Miltenyi Biotec, 130-017-601 (5 µL per 1 × 10⁷ cells)), plus CD34 beads (e.g., CliniMACS CD34, Miltenyi Biotec, 130-017-501 (2.5 µL per 1 × 10⁷ cells)) in the case that a frozen apheresis blood product is used, are added and suspended, and the resultant is incubated at 4°C for 15 minutes, to which separation buffer (e.g., PBS containing 0.5% human type AB serum (inactivated at 56°C for 30 minutes) and 2 mM EDTA) is added, and the resultant is suspended well and centrifuged; the supernatant is removed, and the cells are suspended in 0.5 mL of separation buffer to reach a cell count up to 1 × 10⁸ cells per LD column (e.g., Miltenyi Biotec, 130-042-901); after adding 2 mL of separation buffer in advance, the cell suspension is added to each of the LD columns, and eluates from the LD columns are collected; to each of the LD columns, 1 mL of separation buffer is further added, and eluates therefrom are collected; and the collected liquid is centrifuged, the supernatant is removed, and the cells are then suspended in a proper medium (e.g., KBM501 containing either 5% human type AB serum (inactivated at 56°C for 30 minutes) or 5% UltraGRO (AventaCell, HPCPLCRL10) with 2U/mL heparin sodium supplemented) at 5 × 10⁵ cells/mL in the case that peripheral blood is used or at 1 × 10⁶ cells/mL in the case that a frozen apheresis blood product is used, and cultured until day 14 with appropriate medium exchange.

Reference can be made to Japanese Patent Laid-Open No. 2021-136883 for a specific production method for the highly active NK cells of [3].

[4] Cells obtained through any one of the methods for obtaining the cells of [1] to [3], wherein the cells are cultured with adding any selected from the group consisting of IL-12, IL-15, and IL-18 simultaneously with or in place of IL-2. Reference can be made to Leong JW et al. Biol Blood Marrow Transplant 20 (2014) 463-473 for a specific production method for such cells.

The surface protein of NK cells to which the monobody of the present invention binds is a protein expressed on the surfaces of NK cells, and may be a receptor or a membrane protein. Such proteins include NKp46, NKp30, NKG2D, IL-15R, IL-2R, KIR3DS1, NKG2C, NKp80, NKp65, NKp44, LALRA1, LILRA2, DNAM-1, and 2B4, and the surface protein is preferably at least one selected from the group consisting of NKp46, NKp30, and 2B4.

NKp46, NKp30, and 2B4 are receptors that regulate cytotoxicity. NKp46 (CD335) and NKp30 (CD337) are each an NK cytotoxicity receptor (Natural Cytotoxicity Receptor: NCR). When a ligand binds to such a receptor, the NK cell is activated through signaling to increase the production of cytokines, and the apoptosis of target cells is induced by the release of perforin and granzyme. NKp46 is associated with hemagglutinin present on the surfaces of viruses as a ligand, and NKp30 is associated with B7-H6 expressed on tumor cells as a ligand. 2B4 (CD244) binds to CD48 expressed on other immunocytes to transduce two signals: activation and inhibition.

The monobody of the present invention may bind to NKp46 if the monobody has an amino acid sequence of any one of (1a) to (3a):
(1a) an amino acid sequence represented by any one of SEQ ID NOs: A1 to A5;
(2a) an amino acid sequence given by deletion, substitution, and/or addition of one or more amino acids in an amino acid sequence represented by any one of SEQ ID NOs: A1 to A5 excluding arbitrary amino acids; and
(3a) an amino acid sequence having an identity of 80% or more with an amino acid sequence represented by any one of SEQ ID NOs: A1 to A5 excluding arbitrary amino acids.

The monobody of the present invention may bind to 2B4 if the monobody has an amino acid sequence of any one of (1b) to (3b):
(1b) an amino acid sequence represented by any one of SEQ ID NOs: A6 to A9;
(2b) an amino acid sequence given by deletion, substitution, and/or addition of one or more amino acids in an amino acid sequence represented by any one of SEQ ID NOs: A6 to A9 excluding arbitrary amino acids; and
(3b) an amino acid sequence having an identity of 80% or more with an amino acid sequence represented by any one of SEQ ID NOs: A6 to A9 excluding arbitrary amino acids.

The monobody of the present invention may bind to NKp30 if the monobody has an amino acid sequence of any one of (1c) to (3c):
(1c) an amino acid sequence represented by any one of SEQ ID NOs: A10 to A12;
(2c) an amino acid sequence given by deletion, substitution, and/or addition of one or more amino acids in an amino acid sequence represented by any one of SEQ ID NOs: A10 to A12 excluding arbitrary amino acids; and
(3c) an amino acid sequence having an identity of 80% or more with an amino acid sequence represented by any one of SEQ ID NOs: A10 to A12 excluding arbitrary amino acids.

The definitions of terms and signs and preferred modes for the amino acid sequences of (1a) to (3a), (1b) to (3b), and (1c) to (3c) are the same as the definitions and preferred modes described above for the amino acid sequences of (1) to (3).

The monobody of the present invention binds to a surface protein of NK cells. Accordingly, each NK cell and any protein can be stably bound together via a crosslinking body including the monobody of the present invention. The protein to be bound to each NK cell via a crosslinking body including the monobody of the present invention is not limited, and is, for example, an antibody, and preferably an antibody drug. The antibody drug may be one for treatment of cancer or an infection.

Thus, the present invention also provides an NK cell, wherein the monobody of the present invention is bound to a surface protein. In this NK cell, the monobody of the present invention is bound to the NK cell through recognition of the surface protein of the NK cell. Any compound may be linked to the monobody of the present invention directly or via a linker, for example, at the C terminus. The compound to be linked to the monobody may be a compound that recognizes a desired protein to be bound to the NK cell or a label for detecting the NK cell. In the case that an antibody is bound to the NK cell, an antibody that binds to the Fc region of that antibody or an antigen-binding fragment of an antibody may be bound to the C terminus of the monobody of the present invention. Antigen-binding fragments of an antibody include a single-chain Fv fragment (scFv), dimeric scFv (di-scFv), a diabody, a triabody, a tetrabody, Fab, F(ab')₂, Fv, and a monobody.

The present invention also provides a pharmaceutical composition containing NK cells to each of which an antibody is bound via a crosslinking body including the monobody of the present invention.

In the pharmaceutical composition, the NK cells to each of which an antibody is bound via a crosslinking body including the monobody of the present invention may be in the form suspended in a solution. The solution to suspend the NK cells may be, for example, a cryoprotective solution containing DMSO, physiological saline, phosphate-buffered saline (PBS), medium, or serum. The solution may contain a medicament and a pharmaceutically acceptable carrier as a quasi drug.

The pharmaceutical composition provided by the present invention can be applied to treatment of various diseases susceptible to highly active NK cells or the like. Examples of such diseases are cancer and infections, and specific examples include, but are not limited to, skin cancer, mouth cancer, gallbladder cancer, bile duct cancer, lung cancer, liver cancer, gastric cancer, colorectal cancer, pancreatic cancer, kidney cancer, ovarian cancer, bladder cancer, prostate cancer, neuroblastoma, and leukemia, and infections with viruses and those with bacteria.

Immunotherapy with the pharmaceutical composition of the present invention may be performed singly, or in combination with, for example, surgical therapy, chemotherapy, radiotherapy, and/or an antibody drug.

The present invention includes as an embodiment:
a treatment method including administering an effective amount of NK cells to each of which an antibody is bound via a crosslinking body including the monobody of the present invention to a patient having any of the above diseases;
an NK cell to which an antibody is bound via a crosslinking body including the monobody of the present invention for use in treating or preventing any of the above diseases;
use of NK cells to each of which an antibody is bound via a crosslinking body including the monobody of the present invention for producing a pharmaceutical composition for use in treating or preventing any of the above diseases; and
a therapeutic or prophylactic agent for any of the above diseases, the agent containing NK cells to each of which an antibody is bound via a crosslinking body including the monobody of the present invention.

Regarding the modes described herein, an arbitrary combination of any of the modes including the modes described herein and modes described herein as preferred modes or the like may be employed as a mode.

### Examples

The following more specifically describes the present invention with examples and comparative examples. The present invention is by no means limited by examples given below.

### [Apparatuses]

In Examples, the following apparatuses were used.
PCR apparatus: BIO-RAD T100 Thermal Cycler
RT-PCR apparatus: Roche Light Cycler 96
Absorbance meter: Thermo Nano Drop 200c
Gel imaging system: BIO-RAD EZ Imager
Gel fluorescence imaging system: BIO RAD Pharos FXTM Molecular Imager (excitation light: 532 nm, detection wavelength: 605 nm: HEX)
Next-generation sequencer: Ion PGM^{™} system
Fluorophotometer: JASCO Spectrofluorometer FP-8500
Protein purification apparatus: NGC ^{™} Chromatography Systems
Dissociation constant measurement: Octet^{®} RED 96

### [Reagents]

Synthetic oligonucleotides (Table 4) and DNA sequence analysis were outsourced to Fasmac Co., Ltd., and Dynabeads ^{™} M-280 Streptavidin, Dynabeads ^{™} M-270 Streptavidin, and Dynabeads ^{™} Protein G were purchased from Thermo Fisher Scientific.

Biotinylated Human NKp46/NCR/CD335 Protein, Fc, Avitag ^{™} (NC1-H82F9), Biotinylated Human 2B4/CD244/SLAMF4 Protein, Fc, Avitag ^{™} (2B4-H82F0), and Human NKp30/NCR3/CD337 Protein, Fc Tag (NC3-H5259), as target proteins, were purchased from ACROBiosystems.

For library mRNAs having sequences shown later, ones prepared in accordance with previous reports were used.

Details of the reagents used in Examples are as follows.
· 5 × PfSH Buffer
   50 mM Tris-HCl pH8.4, 500 mM KCI, 0.5% (v/v) Triton^{®} X-100, 10 mM MgSO₄
· HsolA
   18.4 mM GTP, 18.4 mM ATP, 9.18 mM CTP, 9.18 mM UTP, 184 mM creatine phosphate, 459 mM HEPES-KOH pH 7.6, 919 mM potassium acetate, 121 mM Mg acetate 4H₂O 18.4 mM spermidine, 9.18 mM DTT, 13.8 mg/mL tRNA mix, 14 mM Mg (OAc)₂
· 3 × RT mix
   3 × RT buffer, 16.5 mM DTT, 15 mM dNTPs, 7.5 µM sAbS.R28, 250 mM MgCl₂, 12% (v/v) HMLV
· PCR Syber
   1× PCR dNTPs, 1/200000 Syber green I Nucleic Acid Gel Stain, 1/6000 PfSH, 0.375 µM T7SD8M2.F44, 0.375 µM RealTime primer, 2% (v/v) DMSO
· 2 × DEP PCR mix
   1 × PCR dNTPs, 1/3000 PfSH, 25 µM each T7SD8M2P.F47, Mos-G5R-T-an21-3.R45, 4% (v/v) DMSO
· HsolB v12.5 (-T7)
   0.73 µM AlaRS, 0.03 µM ArgRS, 0.38 µM AsnRS, 0.13 µM AspRS, 0.02 µM CysRS, 0.06 µM GlnRS, 0.23 µM GluRS, 0.09 µM GlyRS, 0.02 µM HisRS, 0.40 µM IleRS, 0.04 µM LeuRS, 0.11 µM LysRS, 0.03 µM MetRS, 0.68 µM PheRS, 0.16 µM ProRS, 0.04 µM SerRS, 0.09 µM ThrRS, 0.03 µM TrpRS, 0.02 µM TyrRS, 0.02 µM ValRS,0.6 µM MTF, 0.26 µM EF-G, 0.25 µM RF2, 0.17 µM RF3, 0.5 µM RRF, 4 µg/mL CK, 0.1 µM Adk, 0.1 µM CiPPase, 0.1 µM NDK, 2.7 µM IF1, 0.4 µM IF2, 1.5 µM IF3, 20 µM EF-Tu/Ts, 0.5 µM DnaJ, 1.2 µM DnaK, 0.5 µM GrpE, 1.2 µM Ribosome, 10 mM Mg (OAc)₂
· HsolB v12.5 (+T7)
   0.69 µM AlaRS, 0.03 µM ArgRS, 0.36 µM AsnRS, 0.12 µM AspRS, 0.019 µM CysRS, 0.06 µM GlnRS, 0.22 µM GluRS, 0.09 µM GlyRS, 0.02 µM HisRS, 0.38 µM IleRS, 0.04 µM LeuRS, 0.10 µM LysRS, 0.03 µM MetRS, 0.64 µM PheRS, 0.15 µM ProRS, 0.04 µM SerRS, 0.09 µM ThrRS, 0.03 µM TrpRS, 0.02 µM TyrRS, 0.02 µM ValRS, 0.57 µM MTF, 0.25 µM EF-G, 0.24 µM RF2, 0.16 µM RF3, 0.47 µM RRF, 3.87 µg/mL CK, 0.09 µM Adk, 0.09 µM CiPPase, 0.09 µM NDK, 2.55 µM IF1, 0.38 µM IF2, 1.4 µM IF3, 18.9 µM EF-Tu/Ts, 0.47 µM DnaJ, 1.13 µM DnaK, 0.47 µM GrpE, 1.13 µM Ribosome, 1 µM T7 RNA Polymerase, 10 mM Mg (OAc)₂
· HBST
   50 mM Hepes-K pH7.5, 300 mM NaCl, 0.05% (v/v) Tween20
· 2 × DNA loading buffer
   50× TAE 0.6 mL, 80 % Glycerol 3.75 mL, ultrapure water 25.65 mL
· 10 × RT buffer
   500 mM Tris-HCl (pH8.4), 30 mM MgCl₂, 750 mM KCI
· 10 × Annealing buffer
   250 µM Hepes-K, 2 M AcOK
· 3 × RT mix (-enzyme, -primer)
   10 × RT buffer (Mg :f.c. 3 mM), 5.5 mM DTT, 0.5 µM dNTPs 18 mM MgCl2 (sel)
· 3 × RT mix (+enzyme, +primer)
   80.55 µL of 3x RT mix (-enzyme -pri), 7.5 µL of 100 µM RT primer, and 12 µL of HMLV Reverse transcriptase (RNaseH-) 1710 were mixed.
· 2× SDS loading buffer
   60 µL of SDS loading buffer, 40 µL of 10% SDS, and 2 µL of 1 M DTT were added and mixed.
· RT mix for 1^{st}
   To 250 µL of 2 M Tris-HCl pH 8.4 (0.1 M rt) (f.c. 46.6 mM), 250 µL of 3 M KCI (f.c. 70 mM), 85.5 µL of 2.5 M MgCl₂ (f.c. 19.9 mM), and 50 µL of 1 M DTT (f.c. 4.67 mM) were added and mixed.
· 1 × IMAC B1 (-Glycerol)
   20 mM Hepes-K pH7.8, 300 mM KOAc, 5 mM Imidazole, 0.2 mM DTT
· 1 × IMAC B1
   20 mM Hepes-K pH7.8, 300 mM KOAc, 5 mM Imidazole, 10% Glycerol, 0.2 mM DTT
· 1 × IMAC B1K
   20 mM Hepes-K pH7.8, 300 mM KOAc, 1 M KCI, 5 mM Imidazole, 10% Glycerol, 0.2 mM DTT
· 1 × IMAC B1K (+ATP)
   20 mM Hepes-K pH7.8, 300 mM KOAc, 1 M KCI, 5 mM Imidazole, 10% Glycerol, 2 mM ATP, 10 mM MgSO₄, 0.2 mM DTT
· 1 × IMAC B3
   500 mM Imidazole pH7.8, 300 mM KOAc, 10% Glycerol, 0.2 mM DTT

### [Method for selecting monobodies that specifically bind to target molecules by TRAP display method]

### 1^{st} round

(Annealing/translation/reverse transcription) An annealing solution was prepared to fit the mRNA concentration of each BC-FG or CD-FG library. The annealing solution was subjected to annealing with the PCR apparatus under conditions of [95°C (3 min) - 25°C (5 min, 0.5°C /sec)]. With this solution, 500 µL of a translation solution [11.3% (v/v) HsolA, 0.5 mM 20aa, 15.4% (v/v) HsolB (-T7), 1 µM mRNA/PuLin-an21 (OMe)] was prepared on iced water. After reaction in a water bath at 37°C for 10 minutes, 41.7 µL of 200 mM EDTA (pH 8.0) was added and mixed well and the resultant was left to stand at room temperature for 3 minutes. To the residual solution, 41.1 µL of RT mix for 1^{st} was added, and 66.7 µL of 5 mM dNTPs, 9 µL of 111 µM Mos-G5R-T.R28, and 27.5 µL (4% v/v) of H-MMLV (RNaseH-, 28.7 µM) were then added and mixed well, and reverse transcription reaction was performed on a heat block at 42°C for 15 minutes.

(Gel filtration) To a 2-mL column (Zeba ^{™} Spin Desalting Columns 7K, MWCO), 690 µL of the solution after the reverse transcription was added, and then centrifuged at 2300 rpm for 2 minutes.

(Negative selection) To a mixture of 200 µL of magnetic beads (Dynabeads ^{™} M-280 Streptavidin) washed with HBST and 100 µL of magnetic beads (Dynabeads ^{™} Protein G) washed with HBST, 690 µL of the solution after the gel filtration was added, and mixed by rotation with a rotator in a thermostatic chamber at 25°C for 10 minutes.

(Positive Selection) The supernatant was collected while the magnetic beads were gathered with a strong magnet, and each target was added and mixed to give a final concentration of 10 nM, and incubated at room temperature for 10 minutes. At that time, preparation was performed in such a manner that the final concentration of each target reached 10 nM. This solution was added to and mixed with 200 µL of magnetic beads (Dynabeads ^{™} M-280 Streptavidin). Only the supernatant was collected, and added to 30 µL of magnetic beads (Dynabeads ^{™} Protein G) washed with HBST. The carrier was washed with 500 µL of HBST. This bead washing operation was repeated one more time, the supernatant was removed, and 690 µL of 1× PCR dNTPs was added to the beads to suspend them, and the resultant was heated at 95°C for 5 minutes. After returning to room temperature, the supernatant was collected, and used as a Positive solution.

(Realtime-PCR) The solution after the gel filtration, diluted by a factor of 1/10000 with 0.5× PfSH, and the Positive solution were each aliquoted into 2-µL portions, 18 µL of PCR Syber (primer: T7SD8M2.F44, MoS-RealTime.R20) was added to each, and cDNA contained in each sample was quantified through Real-time PCR under 94°C (15 sec) - [94°C (15 sec) - 60°C (15 sec) - 72°C (30 sec)] × 40 cycles.

(PCR) To 675 µL of the Positive solution, the same amount of 2 × DEP PCR mix was added. The resultant was aliquoted into 20 portions each in 70 µL on ice, and PCR was performed under conditions of 94°C (1 min) - [94°C (20 sec) - 65°C (20 sec) - 72°C (45 sec)] × 12 cycles. ΦOH/CHCl₃ treatment and iPrOH precipitation (0.81 vol) were performed. The precipitate was dissolved in 135 µL of 10 mM Tris-AcOH pH 7.8, and the resultant was used as 10× 1^{st} DNA.

### Transcription for 2^{nd} round

(Transcription) To a mixture of 10 µL of 10× T7 Buffer, 1 µL of 1 M DTT, 1 µL of 2.5 M MgCl₂, 20 µL of 25 mM NTPs, 1.5 µL of 2 N KOH, and 39.8 µL of ultrapure water, and then 20 µL of the 10× 1^{st} DNA and 2.5 µL of 1 µM T7 RNA polymerase were added. The resultant was reacted in a thermostatic chamber at 37°C for 4.5 h. To 95.8 µL of the sample after the transcription, 6 µL of 0.5 M EDTA pH 8.0 was added, and 100 µL of 0.6 M NaCl was then added. ΦOH/CHCl₃ treatment and iPrOH precipitation (0.75 vol) were performed. The precipitate was dissolved in 10 µL of ultrapure water, and the resultant was used as 10× 1^{st} mRNA. After UV measurement to determine the concentration, 9.6 µM 1^{st} mRNA was prepared with ultrapure water. The 2^{nd} round was performed in the same manner as the 1^{st} round, with the reaction scale reduced to 7 µL. However, transcription of RNA was not performed.

### 3^{rd} round

(Transcription/annealing/translation/reverse transcription) On iced water, 35 µL of a translation solution [11.3% (v/v) HsolA, 0.5 mM 20aa, 20 µM PuLin-an21-3 (OMe), 16.7% (v/v) HsolB (+T7), 25 nM 2^{nd} DNA] was prepared. Incubation in a water bath at 37°C for 30 minutes, and then transcription, annealing, and translation reactions were performed. The translation reaction was terminated by adding 6.8 µL of 100 mM EDTA (pH 8.0). A 1/2 (v/v) volume of 3× RT mix (+enzyme, +primer: Mos G5R-T.R28) was added, and the resultant was incubated at 42°C for 15 minutes.

(Gel filtration) Into a 500-µL column (Zeba ^{™} Spin Desalting Columns 7K, MWCO), 61.2 µL of the reverse transcription solution was put, 15 µL of HBST was then added from the top, and the column was centrifuged at 1500 g for 2 minutes.

(Negative selection) Negative selection was performed three times with 55 µL of magnetic beads (50% each Dynabeads ^{™} M-270 + Biotinylated Human IgG1 Fc protein, Avitag ^{™} / Dynabeads ^{™} M-280).

(Positive Selection-Nkp46,2B4) To 92.4 µL of the solution, 112 nM Fc was added. Therefrom, 9-µL portions were taken, 1 µL of each target at 100 nM was added, and the resultant was incubated in a thermostatic chamber at 25°C for 5 minutes (target concentration: 10 nM). This solution was added to 0.5 µL of magnetic beads (Dynabeads ^{™} M-270 Streptavidin) washed with HBST, and the magnets were washed twice with HBST. The beads were suspended by adding 40 µL of 1× PCR dNTPs, and heated at 95°C for 5 minutes. After returning to room temperature, the supernatant was collected, and used as a Positive solution.

(Positive Selection-Nkp30) To 16.8 µL of the solution, 2.11 µL of HBST was added. Thereafter, 9-µL portions were taken therefrom, 1 µL of each target at 100 nM was added, and the resultant was incubated in a thermostatic chamber at 25°C for 5 minutes. This solution was added to 0.5 µL of magnetic beads (Dynabeads ^{™} Protein G) washed with HBST, and the magnets were washed twice with HBST. The beads after removing the supernatant were suspended by adding 40 µL of 1× PCR dNTPs, and heated at 95°C for 5 minutes. After returning to room temperature, the supernatant was collected, and used as a Positive solution. Realtime-PCR and PCR were performed in the same manner as the 2^{nd} round.

In 4^{th} and subsequent rounds, experiment was performed in the same manner with the reaction scale set to 5 µL. In 6th and subsequent rounds, the concentration of each target was set to 1 nM, and, when a bindable monobody was obtained, selection operations were performed under stricter conditions for washing beads. The obtained DNAs were analyzed with the next-generation sequencer.

### Cloning

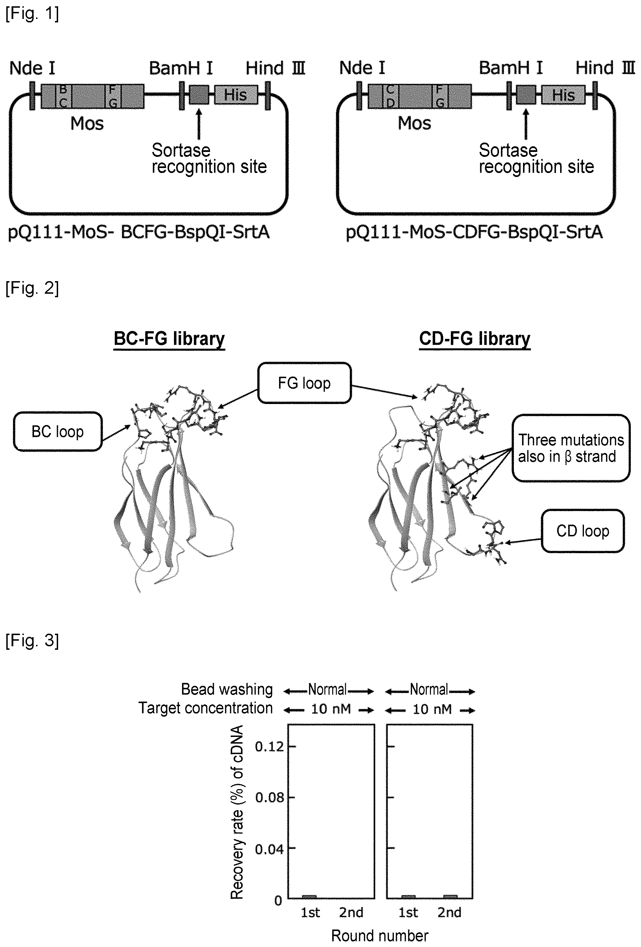

Expression vectors shown in Figure 1 were constructed with an MuPaC method (Taniguchi, N.; Nakayama, S.; Kawakami, T.; Murakami, H., Patch cloning method for multiple site-directed and saturation mutagenesis. BMC Biotechnol. 2013, 13: 91.).

### Expression and purification of monobodies

BL21 (DE3) LysS was transformed with each of the obtained plasmids, and expression was performed with 0.5 mM IPTG at 25°C. To the bacterial cells, 30 mL of an eluent solution was added, the bacterial cells were ultrasonically crushed on iced water, 1.8 mL of 80% glycerol was added to the homogenate solution, and the resultant was centrifuged at 4°C and 13000 rpm for 10 minutes. Filtration was performed, and the resultant was purified with NGC ^{™} Chromatography Systems (Bio-Rad Laboratories, Inc.).

### Evaluation of monobodies by dissociation constant measurement

The binding abilities and specificities were examined by using an Octet ^{®} RED96 (ForteBio) with bio-layer interferometry. Measurement was performed with the following program: Dip for 50 seconds, Load for x seconds (until a signal of 1.0 nm (NKp46, 2B4) or 0.8 nm (Nkp30) was reached), Baseline for 60 seconds, Association for 600 seconds, and Dissociation for 600 seconds. At that time, the monobody concentrations were equally set to 40 nM. The acquired data were analyzed with ForteBio Data Analysis 10.0. Only samples for which binding ability and specificity were found were subjected to measurement of dissociation constants under different conditions. The measurement conditions are shown in a table given below. Dip, Baseline, Association, and Dissociation were performed for the same times as in examining binding ability and specificity. The monobodies were serially diluted from the highest concentration by a factor of 2 to prepare five concentrations for use in measurement.

**[Table 1]**

| Table 1: Conditions for measurement of dissociation constants | | |
|---|---|---|
| Measurement condition | Signal at Loading (nm) | Monobody concentration (nM) |
| Condition 1 | 0.8 | 20-1.25 |
| Condition 2 | 0.8 | 10-0.625 |
| Condition 3 | 0.6 | 10-0.625 |

### [Example 1: Selection using monobody libraries]

The sequence of a human fibronectin type III domain (FN3) (PDB: 1FNA) as a scaffold (skeleton) was partially randomized to prepare monobody libraries, and screening was performed for monobodies specifically bindable to three proteins (NKp46, 2B4, NKp30) on NK cells with a TRAP display method (Transcription-translation coupled with Association of Puromycin-linker display). The human fibronectin type III domain has seven β strands, and six loops connecting each β strand to another are present. In the present example, a BC library with modification of the BC loop and the FG loop and a CD library with modification of the CD loop and the FG loop were prepared. The libraries are shown in Figure 2.

The mRNA sequences of the libraries used and the amino acid sequences after translation are shown in a table given below. ATG in bold characters indicates a start codon, each underlined part indicates an insertion sequence, each underlined part in bold characters indicates a flexible linker sequence, each part with shading indicates a random part, and each numerical subscript indicates the number of randomized amino acids. The random parts were designed to include 20% Tyr, 10% Ser, 10% Gly, 15% Trp, and 3% other amino acids except Cys.

**[Table 2]**

| | |
|---|---|
| mRNA sequence of BC-FG library | |
| GGATTAAGGAGGTGATATTTATGCCTAATCGTCTAGAAGTTGTTGA AGCGTCCCCCACCAGCATTCAGATCAGCTGGGATGCT(NNK)6-12GT GCGTTATTACCGTATCACTTACGGAGAAACAGGAGGAAATAGCCC TGTCCAGGAGTTCACTGTGCCTGGGAGCAAGTCTACAGCTACCAT CAGCGGCCTTAAACCTGGAGTTGATTATACCATCACTGTGTATGCT GTCACT(NNK)10-18CCAATTTCCATTAATTACCGTACAGAAATTGACA AACCAAGTCAAGGTGGAGGAGGAGGCCGATAGTGGACGGCGGG CGCGAGGCGGG | (SEQ ID NO: 49) |
| Amino acid sequence of BC-FG after translation | |
| MPNRLEVVEASPTSIQISWDAX6-12VRYYRITYGETGGNSPVQEFTVP GSKSTATISGLKPGVDYTITVYAVTX10-18PISINYRTEIDKPSQGGGGG R | (SEQ ID NO: 50) |
| mRNA sequence of CD-FG library | |
| GGATTAAGGAGGTGATATTTATGCCTAATCGTCTAGAAGTTGTTG AAGCGTCCCCCACCAGCATTCAGATCAGCTGGGATGCTCCTGCT GTCACAGTGCGTTATTAC(NNK)ATC(NNK)TACGGAGAAACAGGA( NNK)9-11GTCCAG(NNK)TTCACTGTGCCTGGGAGCAAGTCTACAGC TACCATCAGCGGCCTTAAACCTGGAGTTGATTATACCATCACTGT GTATGCTGTCACT(NNK)10- 18CCAATTTCCATTAATTACCGTACAGA AATTGACAAACCAAGTCAAGGTGGAGGAGGAGGCCGATAGTGG ACGGCGGGCGCGAGGCGGG | (SEQ ID NO: 51) |
| Amino acid sequence of CD-FG after translation | |
| MPNRLEVVEASPTSIQISWDAPAVTVRYYXIXYGETGX9-11VQXFTVP GSKSTATISGLKPGVDYTITVYAVTX10-18PISINYRTEIDKPSQGGGGG R | (SEQ ID NO: 52) |

The primers used are shown in the following.

**[Table 3]**

| Table 3: List of synthetic oligonucleotides (5' -> 3') | | |
|---|---|---|
| Primer name | Sequence | SEQ ID NO: |
| TRAP display method | | |
| T7SD8M2.F44 | ATACTAATACGACTCACTATAGGATTAAGGAGGTGAT ATTTATG | 53 |
| MoS-RealTime.R20 | AGCATCCCAGCTGATCTGAA | 54 |
| Mos G5R-T.R28 | ACTATCGGCCTCCTCCTCCACCTTGACT | 55 |
| T7SD8M2P.F47 | ATACTAATACGACTCACTATAGGATTAAGGAGGTGAT ATTTATGCCT | 56 |
| Mos-G5R-T-an21-3.R45 | CCCGCCTCGCGCCCGCCGTCCACTATCGGCCTCC TCCTCCACCTT | 57 |

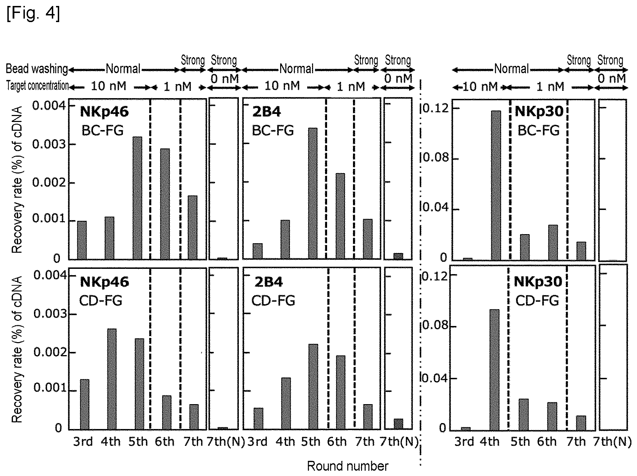

The protein diversity was 2.6 × 10¹³ for the BC-FG library and 4.4 × 10¹³ for the CD-FG library. Figures 3 and 4 show recovery rates of cDNA obtained with the TRAP display method in each round for each target. Each recovery rate was calculated by dividing the amount of recovered cDNA by the amount of the puromycin linker contained in the system. Selection was performed for a mixture of targets in the 1^{st} round and the 2^{nd} round, and separately for each target in the subsequent rounds. Each target concentration was first set to 10 nM, and reduced to 1 nM when a significantly increased recovery rate was found. After that, selection under stronger conditions for washing magnetic beads was performed to obtain a monobody having lower k_{off}. For the results for each target, the rightmost blue graph shows the recovery rate in Negative selection without binding of the target to beads, and the orange graphs each show the recovery rate in Positive selection with binding of the target to beads. Figure 4 demonstrates that at the 7^{th} round the recovery rate in Positive selection was higher than the recovery rate in Negative selection for any of the targets, and thus it is understood that monobodies bindable to the targets of interest were successfully obtained.

### [Example 2: Sequence analysis with next-generation sequencer]

cDNAs recovered in Positive selection and Negative selection at the 7^{th} round were analyzed with the next-generation sequencer, and some monobodies were then selected. Tables 4 to 6 show the selected monobodies.

In the case of targeting NKp46, three monobodies having P/N ratios of 3.0 or more were selected for the BC-FG library. The total number of reads was 370854. For the CD-FG library, two monobodies having P/N ratios of 5.0 or more were selected. The total number of reads was 251088.

In the case of targeting 2B4, two monobodies having P/N ratios of 4.0 or more were selected for the BC-FG library. The total number of reads was 502168. For the CD-FG library, two monobodies having P/N ratios of 2.5 or more were selected. The total number of reads was 237279.

In the case of targeting NKp30, two monobodies having P/N ratios of 5.0 or more were selected for the BC-FG library. The total number of reads was 424980. For the CD-FG library, one monobody having a P/N ratio of 4.0 or more was selected. The total number of reads was 256719.

**[Table 4]**

| Table 4: Results of sequence analysis with next-generation sequencer for monobody libraries targeting NKp46 (BC-FG: top, CD-FG: bottom) | | | | | | |
|---|---|---|---|---|---|---|
| Clone | Number of reads | BC loop | FG loop | Appearance rate (%) | P/N ratio | P/N ratio' (×10) |
| Mb-NKp46-BC-1 | 16404 | PIWGRYGT | TVRGQTYYWWML | 4.4 | 10.0 | 42 |
| Mb-NKp46-BC-2 | 12695 | WTWLGYEYYW | WDSKPGAIGI | 3.4 | 11.2 | 47 |
| Mb-NKp46-BC-3 | 3838 | TYWDWGFR | WSWARRTHISWF | 1.0 | 3.7 | 16 |

| Clone | Number of reads | CD loop | | | | FG loop | Appearance rate (%) | P/N ratio | P/N ratio' (×10) |
|---|---|---|---|---|---|---|---|---|---|
| Mb-NKp46-CD-1 | 12266 | D | Y | PGYYMWQRGVIN | A | GQERAYGHPEHF | 4.9 | 8.3 | 13 |
| Mb-NKp46-CD-3 | 7548 | V | S | RYKYHRRIN | G | GTQWYVFNEAGDVAGY | 3.0 | 5.9 | 9.3 |

**[Table 5]**

| Table 5: Results of sequence analysis with next-generation sequencer for monobody libraries targeting 2B4 (BC-FG: top, CD-FG: bottom) | | | | | | |
|---|---|---|---|---|---|---|
| Clone | Number of reads | BC loop | FG loop | Appearance rate (%) | P/N ratio | P/N ratio' |
| Mb-2B4-BC-1 | 34498 | GPIVSYSWWYHW | VGYVNEKYDAWQSYVQ | 6.9 | 6.6 | 46 |
| Mb-2B4-BC-2 | 22125 | QAYWFEYTWW | LEWVYDDQWTSG | 4.4 | 5.5 | 39 |

| Clone | Number of reads | CD loop | | | | FG loop | Appearance rate (%) | P/N ratio | P/N ratio' |
|---|---|---|---|---|---|---|---|---|---|
| Mb-2B4-CD-1 | 23635 | G | Y | TSPWVYNGY | E | DQYKSWNLEEFH | 100 | 20 | 4.9 |
| Mb-2B4-CD-3 | 13349 | E | L | TPPWIHKGW | Y | VTPYGRKWVGMNGW | 5.6 | 2.5 | 6.1 |

**[Table 6]**

| Table 6: Results of sequence analysis with next-generation sequencer for monobody libraries targeting NKp30 (BC-FG: top, CD-FG: bottom) | | | | | | |
|---|---|---|---|---|---|---|
| Clone | Number of reads | BC loop | FG loop | Appearance rate (%) | P/N ratio | P/N ratio' (×10⁴) |
| Mb-NKp30-BC-2 | 9527 | LWHYYGDQYW | WMYVPEYYFNPW | 2.2 | 5.3 | 1.2 |
| Mb-NKp30-BC-6 | 1948 | TDRNYDITYGMQ | YWWDGYSYSEEAWYY | 0.5 | 9.5 | 2.2 |

| Clone | Number of reads | CD loop | | | | FG loop | Appearance rate (%) | P/N ratio | P/N ratio' (×10⁴) |
|---|---|---|---|---|---|---|---|---|---|
| Mb-NKp30-CD-9 | 1376 | V | T | WSWESYWHGTY | Q | TFMKDIPSYY | 0.5 | 4.2 | 0.5 |

Each P/N ratio in the tables was calculated by dividing the appearance rate of the corresponding sequence in the Positive selection solution by the appearance rate in the Negative selection solution, and lower P/N ratios are expected to result in higher probabilities of the presence of nonspecific binding. Each P/N ratio' was calculated by dividing the recovery rate in Positive selection by the recovery rate in Negative selection and multiplying the resulting value by the P/N ratio.

### [Example 3: Expression and purification]

The monobodies selected in Example 2 were each fused with a Sortase A recognition site and a His tag and expressed, and the products were purified with NGC ^{™} Chromatography Systems. SDS-PAGE confirmed the successful purification. Table 7 shows the primers used for cloning of the monobodies. Table 8 shows a list of yields of clones.

**[Table 7-1]**

| Table 7: List of synthetic oligonucleotides (5' -> 3') | | |
|---|---|---|
| Primer name | Sequence | SEQ ID NO: |
| Cloning of monobody | | |
| Mb-NKp46- BC-1 F40 | TTCAGATCAGCTGGGATGCTCCGATCTGGGGTCGTT ACGG | 58 |
| Mb-NKp46- BC-2 F40 | TTCAGATCAGCTGGGATGCTTGGACTTGGCTCGGTT ACGA | 59 |
| Mb-NKp46- BC-3 F44 | TTCAGATCAGCTGGGATGCTACTTACTGGGACTGGG GTTTCCGT | 60 |
| Mb-2B4-BC-1 F40 | TTCAGATCAGCTGGGATGCTGGTCCGATCGTTTCTTA CTC | 61 |
| Mb-2B4-BC-2 F40 | TTCAGATCAGCTGGGATGCTCAGGCTTACTGGTTCG AATA | 62 |
| Mb-NKp30- BC-2 F40 | TTCAGATCAGCTGGGATGCTCTGTGGCATTACTACGG TGA | 63 |
| Mb-NKp30- BC-6 F40 | TTCAGATCAGCTGGGATGCTACTGACCGTAACTACGA CAT | 64 |
| Mb-NKp46- CD-1 F65 | GCTGTCACAGTGCGTTATTACGACATCTACTACGGAG AAACAGGACCGGGTTACTACATGTGGCA | 65 |
| Mb-NKp46- CD-3 F65 | GCTGTCACAGTGCGTTATTACGTTATCTCTTACGGAG AAACAGGACGTTACAAATACCATCGTCG | 66 |
| Mb-2B4-CD-1 F65 | GCTGTCACAGTGCGTTATTACGGTATCTACTACGGAG AAACAGGAACTTCTCCGTGGGTTTACAA | 67 |
| Mb-2B4-CD-3 F65 | GCTGTCACAGTGCGTTATTACGAAATCCTGTACGGAG AAACAGGAACTCCGCCGTGGATCCATAA | 68 |

**[Table 7-2]**

| Primer name | Sequence | SEQ ID NO: |
|---|---|---|
| Mb-NKp30- CD-9 F78 | GCTGTCACAGTGCGTTATTACGTTATCACTTACGGAG AAACAGGATGGTCTTGGGAATCTTACTGGCATGGTAC TTAC | 69 |
| Mb-NKp46- BC-1 R40 | CGGTAATTAATGGAAATTGGCAGCATCCACCAGTAGT AAG | 70 |
| Mb-NKp46- BC-2 R40 | CGGTAATTAATGGAAATTGGGATACCGATAGCACCCG GTT | 71 |
| Mb-NKp46- BC-3 R40 | CGGTAATTAATGGAAATTGGGAACCAAGAGATATGAG TAC | 72 |
| Mb-2B4-BC-1 R40 | CGGTAATTAATGGAAATTGGCTGAACGTAAGACTGCC AAG | 73 |
| Mb-2B4-BC-2 R40 | CGGTAATTAATGGAAATTGGACCAGAAGTCCACTGGT CGT | 74 |
| Mb-NKp30- BC-2 R40 | CGGTAATTAATGGAAATTGGCCACGGGTTGAAGTAGT ATT | 75 |
| Mb-NKp30- BC-6 R41 | CGGTAATTAATGGAAATTGGGTAGTACCAAGCTTCTT CAGA | 76 |
| Mb-NKp46- CD-1 R40 | CGGTAATTAATGGAAATTGGGAAATGTTCCGGATGAC CGT | 77 |
| Mb-NKp46- CD-3 R40 | CGGTAATTAATGGAAATTGGGTAACCAGCAACGTCAC CAG | 78 |
| Mb-2B4-CD-1 R40 | CGGTAATTAATGGAAATTGGATGGAATTCTTCCAGGT TCC | 79 |
| Mb-2B4-CD-3 R40 | CGGTAATTAATGGAAATTGGCCAACCGTTCATACCAA CCC | 80 |
| Mb-NKp30- CD-9 R50 | CGGTAATTAATGGAAATTGGGTAGTAAGACGGGATGT CTTTCATGAAAGT | 81 |

**[Table 8]**

| Table 8: List of yields of clones | | | | | | | | | | | | |
|---|---|---|---|---|---|---|---|---|---|---|---|---|
| Target | N Kp46 | | | | | 2B4 | | | | NKp30 | | |
| Library | BC-FG | | | CD-FG | | BC-FG | | CD-FG | | BC-FG | | CD-FG |
| Clone | 1 | 2 | 3 | 1 | 3 | 1 | 2 | 1 | 3 | 2 | 6 | 9 |
| Yield (mg) | 1.5 | 1.3 | 0.5 | 3.4 | 1.2 | 0.5 | 1.8 | 2.2 | 1.5 | 1.3 | 3.0 | 2.1 |

### [Example 4: Dissociation constant measurement by bio-layer interferometry]

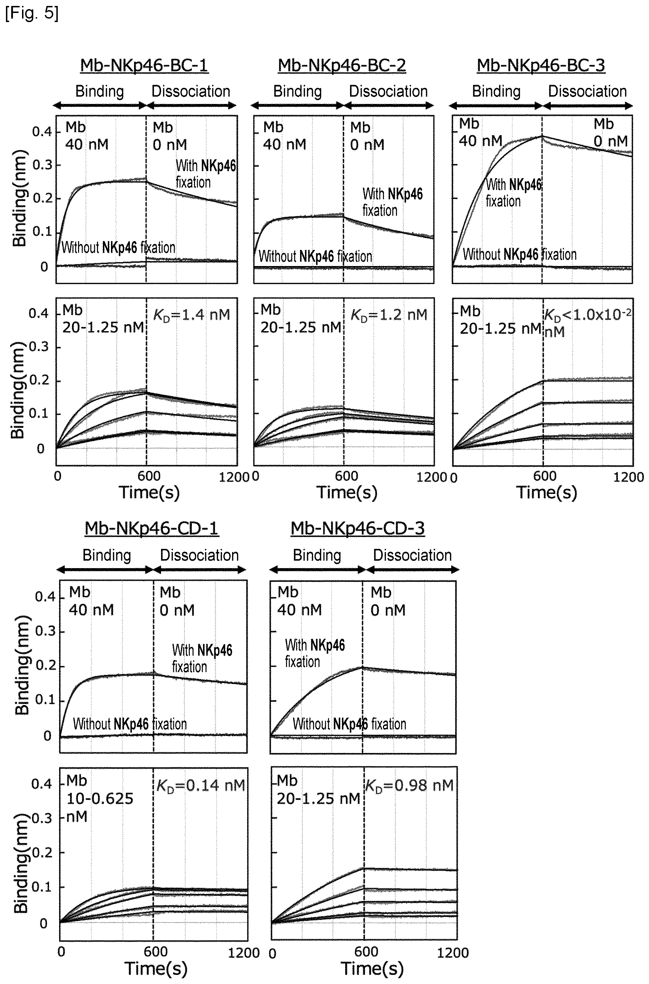

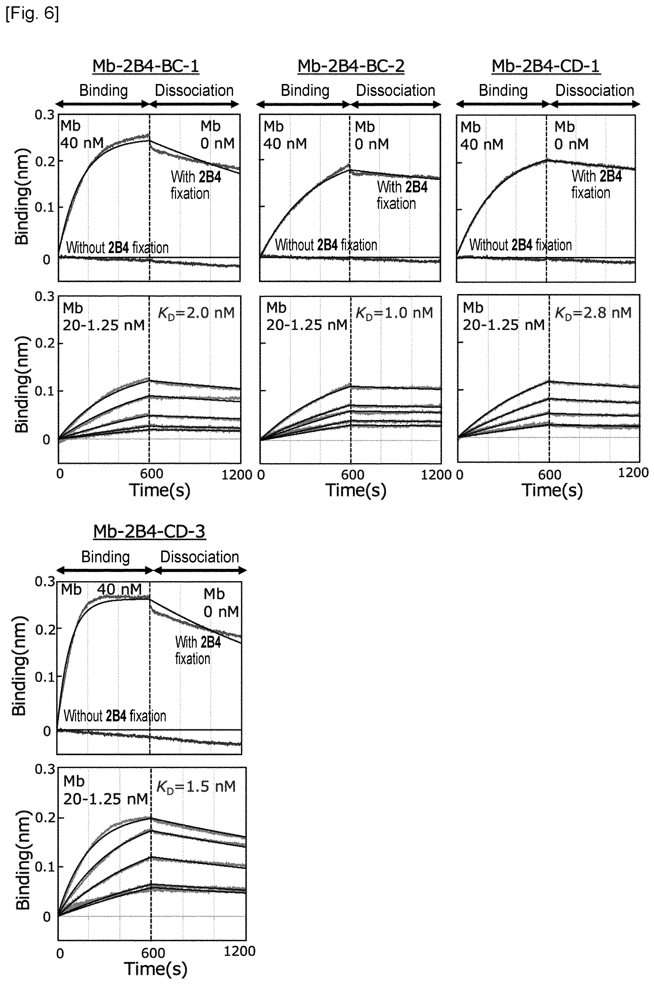

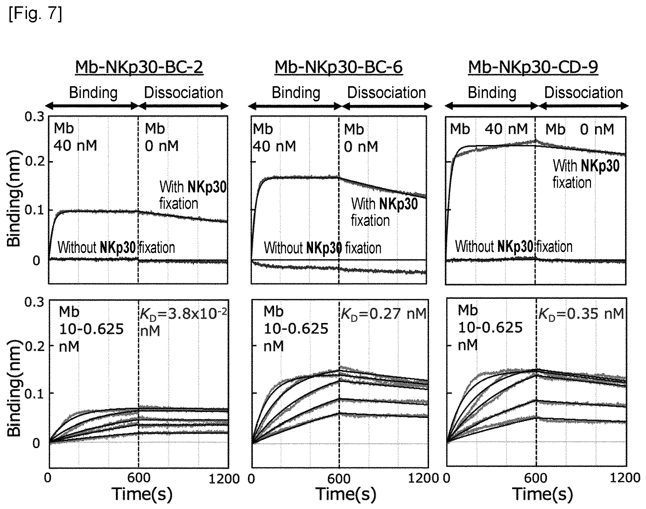

The binding abilities and specificities of all the clones expressed and purified in Example 3 were examined, and the values of dissociation constants were measured. Figures 5 to 7 show the measurement results. In the top graphs in each of Figures 5 to 7, upper lines show results of measurement of the binding abilities of the monobodies with binding of a target to the tip of the sensor, and lower lines show results of examination on whether a monobody was bound only to the sensor without binding of a target to the sensor. Mb in the graphs is an abbreviation for monobody concentration. For any of the clones, the monobody was bound to the sensor when a target was fixed on the sensor, and no binding was found when a target was not fixed on the sensor. The bottom graphs in each of Figures 5 to 7 show results of measurement of dissociation constants for five concentrations.

The following table show results of evaluation of the clones on the presence or absence of binding ability and specificity, etc.

**[Table 9]**

| Table 9: Summary of results of clone binding | | | | | | |
|---|---|---|---|---|---|---|
| Target | Library | Clone | Dissociation constant (nM) | *k*ₒₙ (×10⁵M⁻¹s⁻¹) | *k*_{off} (×10⁻⁴s⁻¹) | Specificity |
| N Kp46 | BC-FG | No. 1 | 1.4 | 3.5 | 5.0 | present |
| | | No. 2 | 1.2 | 3.8 | 4.7 | present |
| | | No. 3 | <1.0×10⁻² | 1.0 | <1.0×10⁻² | present |
| | CD-FG | No. 1 | 0.14 | 5.3 | 0.72 | present |
| | | No. 3 | 0.98 | 0.72 | 0.70 | present |
| 2B4 | BC-FG | No. 1 | 2.0 | 1.2 | 2.5 | present |
| | | No. 2 | 1.0 | 0.92 | 0.93 | present |
| | CD-FG | No. 1 | 2.8 | 0.71 | 2.0 | present |
| | | No. 3 | 1.5 | 2.3 | 3.5 | present |
| NKp30 | BC-FG | No. 2 | 3.8×10⁻² | 7.2 | 0.27 | present |
| | | No. 6 | 0.27 | 9.0 | 2.4 | present |
| | CD-FG | No. 9 | 0.35 | 8.1 | 2.9 | present |

As described hereinbefore, selection of monobodies bindable to a protein on NK cells was performed by means of the TRAP display method with two libraries, BC-FG and CD-FG libraries, obtained by randomizing two loops. The result that at the 7^{th} round the recovery rates in Positive selection with a target fixed to magnetic beads were higher than the recovery rates in Negative selection without a target fixed to magnetic beads found that monobodies bindable to the targets of interest were successfully obtained. Thereafter, cDNAs obtained in Positive selection and Negative selection at the 7^{th} round were analyzed with the next-generation sequencer, and clones to be expressed were selected on the basis of the appearance rates and P/N ratios. Concentration of monobodies was confirmed from the appearance rates. In addition, dissociation constant measurement was performed with the expressed and purified clones by means of the bio-layer interferometry, and monobodies that exhibited strong binding were successfully selected (K_{D} = 1.0 × 10⁻² nM to 2.8 nM). The dissociation constants are almost comparable to those of antibodies that are used for antibody drugs, and hence considered to be values of dissociation constants sufficient for use. k_{off} was as low as 1.0 × 10⁻⁶ s⁻¹ to 5.0 × 10⁻⁴ s⁻¹, and hence it can be said that monobodies of interest that bind to a protein on NK cells and are difficult to dissociate were successfully created.

## Claims

1. A monobody that binds to a surface protein of an NK cell, wherein the monobody has an amino acid sequence of any one of (1) to (3):
(1) an amino acid sequence represented by any one of SEQ ID NOs: A1 to A12;
(2) an amino acid sequence given by deletion, substitution, and/or addition of one or more amino acids in an amino acid sequence represented by any one of SEQ ID NOs: A1 to A12 excluding arbitrary amino acids; and
(3) an amino acid sequence having an identity of 80% or more with an amino acid sequence represented by any one of SEQ ID NOs: A1 to A12 excluding arbitrary amino acids, wherein
X¹, X², X³, X⁴, X⁵, and X⁶ in SEQ ID NOs: A1 to A12 each independently represent an arbitrary amino acid, and n1, n2, n3, n4, n5, and n6 each independently represent an arbitrary natural number.
| | | | | | |
|---|---|---|---|---|---|
| X¹ₙ₁ | PIWGRYGT | X²ₙ₂ | TVRGQTYYWWML | X³ₙ₃ | (SEQ ID NO: A1) |
| X¹ₙ₁ | WTWLGYEYYW | X²ₙ₂ | WDSKPGAIGI | X³ₙ₃ | (SEQ ID NO: A2) |
| X¹ₙ₁ | TYWDWGFR | X²ₙ₂ | WSWARRTHISWF | X³ₙ₃ | (SEQ ID NO: A3) |
| X⁴ₙ₄ | PGYYMWQRGVIN | X⁵ₙ₅ | GQERAYGHPEHF | x⁶ₙ₆ | (SEQ ID NO: A4) |
| X⁴ₙ₄ | RYKYHRRIN | X⁵ₙ₅ | GTQWYVFNEAGDVAGY | X⁶ₙ₆ | (SEQ ID NO: A5) |
| X¹ₙ₁ | GPIVSYSWWYHW | X²ₙ₂ | VGYVNEKYDAWQSYVQ | X³ₙ₃ | (SEQ ID NO: A6) |
| X¹ₙ₁ | QAYWFEYTWW | X²ₙ₂ | LEWVYDDQWTSG | X³ₙ₃ | (SEQ ID NO: A7) |
| X⁴ₙ₄ | TSPWVYNGY | X⁵ₙ₅ | DQYKSWNLEEFH | x⁶ₙ₆ | (SEQ ID NO: A8) |
| X⁴ₙ₄ | TPPWIHKGW | X⁵ₙ₅ | VTPYGRKWVGMNGW | X⁶ₙ₆ | (SEQ ID NO: A9) |
| X¹ₙ₁ | LWHYYGDQYW | X²ₙ₂ | WMYVPEYYFNPW | X³ₙ₃ | (SEQ ID NO: A10) |
| X¹ₙ₁ | TDRNYDITYGMQ | X²ₙ₂ | YWWDGYSYSEEAWYY | X³ₙ₃ | (SEQ ID NO: A11) |
| X⁴ₙ₄ | WSWESYWHGTY | X⁵ₙ₅ | TFMKDIPSYY | x⁶ₙ₆ | (SEQ ID NO: A12) |

2. The monobody according to claim 1, wherein the surface protein is at least one selected from the group consisting of NKp46, NKp30, and 2B4.

3. The monobody according to claim 1, wherein, in the amino acid sequences represented by SEQ ID NOs: A1 to A12,
amino acid sequences represented by SEQ ID NOs: 1 to 12 are each present in a BC loop or CD loop, and
amino acid sequences represented by SEQ ID NOs: 13 to 24 are each present in an FG loop.
| | |
|---|---|
| PIWGRYGT | (SEQ ID NO: 1) |
| WTWLGYEYYW | (SEQ ID NO: 2) |
| TYWDWGFR | (SEQ ID NO: 3) |
| PGYYMWQRGVIN | (SEQ ID NO: 4) |
| RYKYHRRIN | (SEQ ID NO: 5) |
| GPIVSYSWWYHW | (SEQ ID NO: 6) |
| QAYWFEYTWW | (SEQ ID NO: 7) |
| TSPWVYNGY | (SEQ ID NO: 8) |
| TPPWIHKGW | (SEQ ID NO: 9) |
| LWHYYGDQYW | (SEQ ID NO: 10) |
| TDRNYDITYGMQ | (SEQ ID NO: 11) |
| WSWESYWHGTY | (SEQ ID NO: 12) |
| TVRGQTYYWWML | (SEQ ID NO: 13) |
| WDSKPGAIGI | (SEQ ID NO: 14) |
| WSWARRTHISWF | (SEQ ID NO: 15) |
| GQERAYGHPEHF | (SEQ ID NO: 16) |
| GTQWYVFNEAGDVAGY | (SEQ ID NO: 17) |
| VGYVNEKYDAWQSYVQ | (SEQ ID NO: 18) |
| LEWVYDDQWTSG | (SEQ ID NO: 19) |
| DQYKSWNLEEFH | (SEQ ID NO: 20) |
| VTPYGRKWVGMNGW | (SEQ ID NO: 21) |
| WMYVPEYYFNPW | (SEQ ID NO: 22) |
| YWWDGYSYSEEAWYY | (SEQ ID NO: 23) |
| TFMKDIPSYY | (SEQ ID NO: 24) |

4. An NK cell, wherein the monobody according to any one of claims 1 to 3 is bound to a surface protein.
